(19) Europäisches Patentamt
European Patent Office
Office européen des brevets

(11) Veröffentlichungsnummer: **0 428 720 B1**

(12) **EUROPÄISCHE PATENTSCHRIFT**

(45) Veröffentlichungstag der Patentschrift: **22.03.95**

(51) Int. Cl.[6]: **C09K 19/34**, C09K 19/30, C09K 19/12, C09K 19/20, C07C 69/75, C07C 69/757, C07C 69/92, C07C 43/225, C07D 213/30, C07C 69/716, C07C 255/37

(21) Anmeldenummer: **90915943.6**

(22) Anmeldetag: **10.05.90**

(86) Internationale Anmeldenummer: **PCT/EP90/00679**

(87) Internationale Veröffentlichungsnummer: **WO 90/13611 (15.11.90 90/26)**

Die Akte enthält technische Angaben, die nach dem Eingang der Anmeldung eingereicht wurden und die nicht in dieser Patentschrift enthalten sind.

# (54) OPTISCH AKTIVE VERBINDUNGEN UND FLÜSSIGKRISTALLINE PHASE.

(30) Priorität: **11.05.89 DE 3915378**
**13.05.89 DE 3915698**
**23.06.89 DE 3920571**
**07.07.89 DE 3922307**
**07.07.89 DE 3922308**
**07.07.89 DE 3922416**
**14.07.89 DE 3923324**

(43) Veröffentlichungstag der Anmeldung: **29.05.91 Patentblatt 91/22**

(45) Bekanntmachung des Hinweises auf die Patenterteilung: **22.03.95 Patentblatt 95/12**

(84) Benannte Vertragsstaaten: **DE GB**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestrasse 34**
**D-6103 Griesheim (DE)**
Erfinder: **GEELHAAR, Thomas**
**Trajanstrasse 12**
**D-6500 Mainz (DE)**
Erfinder: **HITTICH, Reinhard**
**Am Kirchberg 11**
**D-6101 Modautal 1 (DE)**
Erfinder: **BARTMANN, Ekkehard**
**Dieburger Weg 12a**
**D-6106 Erzhausen (DE)**
Erfinder: **KRAUSE, Joachim**
**Samuel-Morse-Strasse 14**
**D-6110 Dieburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 428 720 B1

(56) Entgegenhaltungen:
EP-A- 0 237 007
EP-A- 0 278 665
EP-A- 0 285 141
EP-A- 0 289 270

Erfinder: **POETSCH, Eike**
**Am Buchwald 4**
**D-6109 Mühltal 9 (DE)**

## Beschreibung

Die Erfindung betrifft optisch aktive Verbindungen der Formel I

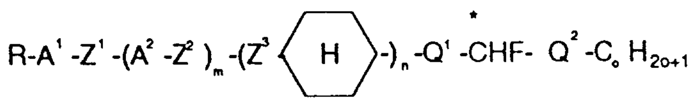

I

worin

| | |
|---|---|
| R | einen unsubstituierten, einen einfach durch -CN oder einen mindestens einfach durch Fluor oder Chlor substituierten Alkyl- oder Alkenylrest mit bis zu 15 C-Atomen, wobei in diesen Resten auch eine $CH_2$-Gruppe durch -O-, -CO-, -O-CO-, -CO-O- oder -O-CO-O- ersetzt sein kann. |
| $A^1$ und $A^2$ | jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, 1,2,4-Thiadiazol-3,5-diyl-Rest, trans-1,4-Cyclohexylen-Rest, worin auch eine oder zwei nicht benachbarte $CH_2$-Gruppen durch -O- und/oder -S-ersetzt sein können und/oder eine CH-Gruppe durch -C(CN)- ersetzt sein kann, |
| $Z^1$, $Z^2$ und $Z^3$ | jeweils unabhängig voneinander -CO-O, -O-CO-, $-CH_2O-$, - $OCH_2$- $-CH_2CH_2-$, -CH=CH-, -C≡C- oder eine Einfachbindung, |
| $Q^1$ | $-CH_2CH_2-$, $-CH_2CH_2CH_2-$, $-OCH_2-$, $CH_2OCH_2-$, - $OCH_2CH_2-$, -O-CO-, |
| $Q^2$ | -O-, -O-CO-, $-(CH_2)_3-O-$ oder eine Einfachbindung, |
| m | 0, 1, 2 oder 3, |
| n | 0 oder 1 und |
| o | 1 bis 9, |

bedeuten,

mit den Maßgaben, daß im Falle $Q^1$ = $-OCH_2-$ oder -O-CO- einer der Ringe $A^1$ und $A^2$ trans-1,4-Cyclohexylen, worin eine CH-Gruppe durch -C(CN)- ersetzt ist, bedeutet.

Die Verbindungen der Formel I können wie ähnliche in DE-OS 35 15 373 beschriebene Verbindungen als Komponenten chiraler getilteter smektischer flüssigkristalliner Phasen verwendet werden.

Ähnliche chirale Verbindungen mit 2-Fluoralkanoyloxy- bzw. 2-Fluoralkoxyresten sind bekannt:
In der EP 0 285 141 werden mesomorphe, chirale 2-Fluoralkanoyloxy-Derivate ($Q^1$ = -O-CO-) und z.B. in der EP 0 278 665, der EP-A- 237 007 und der EP-A- 289 270 mesomorphe, chirale 2-Fluoralkoxy-Derivate ($Q^1$ = $O-CH_2-$) als Komponenten ferroelektrischer Zusammensetzungen beschrieben.

Es gibt dort keinerlei Hinweise darauf, daß Verbindungen, welche Cyclohexanringe aufweisen, worin eine CH-Gruppe durch -C(CN)- ersetzt ist, zu ferroelektrischen Zusammensetzungen mit höheren Werten der Spontanpolarisation und kürzeren Schaltzeiten führen.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Lig. Cryst. 89, 327 (1982); H.R. Brand et al., J. Physique 44, (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 $\mu$m) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc*) ist deren relativ hohe optische Anisotropie, die durch relativ hohe Viskositätswerte bedingten nicht ausreichend kurzen Schaltzeiten, sowie, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3).

Es wurde nun gefunden, daß die Verwendung von Verbindungen der Formel I als Komponenten chiraler getilteter smektischer Mischungen die erwähnten Nachteile wesentlich vermindern kann. Die Verbindungen der Formel I sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc*-Phasenbereichen, negativer oder auch positiver dielektrischer Anisotropie, niedriger optischer Anisotropie, günstiger Pitchhöhe, niedriger Viskosität und für derartige Phasen hohen Werten für die spontane Polarisation und sehr kurzen Schaltzeiten herstellbar. P ist die spontane Polarisation in $nC/cm^2$.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und weisen günstige Werte der optischen Anisotropie auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel I als Komponenten chiraler getilteter smektischer Phasen vorteilhaft eingesetzt werden. Chemisch, thermisch und gegen Licht sind sie sehr stabil.

Gegenstand der Erfindung sind somit die Verbindungen der Formel I sowie die Verwendung der Verbindungen der Formel I als Komponenten flüssigkristalliner Phasen.

Gegenstand der Erfindung sind auch chirale getiltete smektische flüssigkristalline Phasen mit einem Gehalt an mindestens einer Verbindung der Formel I mit mindestens einem mit vier verschiedenen Substituenten verknüpften Kohlenstoffatom.

Gegenstand der Erfindung sind ferner solche Phasen mit einem Gehalt an mindestens einer Verbindung der Formel I sowie Flüssigkristallanzeigeelemente, insbesondere elektrooptische Anzeigeelemente, die derartige Phasen enthalten.

Der Einfachheit halber bedeuten im folgenden Ph eine 1,4-Phenylengruppe, worin auch eine oder zwei CH-Gruppen durch N ersetzt sein können, Cy eine 1,4-Cyclohexylengruppe, worin auch eine oder zwei nicht benachbarte $CH_2$-Gruppen durch O-Atome ersetzt sein können, Dio eine 1,3-Dioxan-2,5-diyl-gruppe, Tia eine 1,3,4-Thiadiazol-2,5-diylgruppe und Bi eine Bicyclo(2,2,2)octylengruppe. Vor- und nachstehend haben R, $A^1$, $Z^1$, $A^2$, $Z^2$, m, $Z^3$, n, $Q^1$ und o $Q^2$ die angegebene Bedeutung, sofern nicht ausdrücklich etwas anderes vermerkt ist.

In den bevorzugten Verbindungen der vor- und nachstehenden Formeln können die Alkylreste, in denen auch eine $CH_2$-Gruppe (Alkoxy bzw. Oxaalkyl) durch ein O-Atom ersetzt sein kann, geradkettig oder verzweigt sein. Vorzugsweise haben sie 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

$A^1$ und $A^2$ sind bevorzugt Cy oder Ph. In den Verbindungen der vor- und nachstehenden Formeln bedeutet Ph vorzugsweise eine 1,4-Phenylen- (Phe), eine Pyrimidin-2,5-diyl-(Pyr), eine Pyridin-2,5-diyl-(Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind. Cy bedeutet vorzugsweise eine 1,4-Cyclohexylengruppe. Insbesondere bevorzugt sind jedoch Verbindungen der Formel I, worin eine der Gruppen $A^2$ eine in 1- oder 4-Position durch CN substituierte 1,4-Cyclohexylengruppe bedeutet und die Nitrilgruppe sich in axialer Position befindet, d.h. eine Gruppe $A^2$, die folgende Konfiguration aufweist:

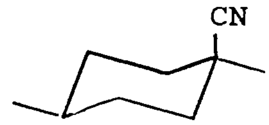

Besonders bevorzugt sind Verbindungen der Formel I und der vorstehenden Teilformeln, die eine Gruppierung -Ph-Ph- enthalten. -Ph-Ph- ist vorzugsweise -Phe-Phe-, Phe-Pyr oder Phe-Pyn. Besonders bevorzugt sind die Gruppen

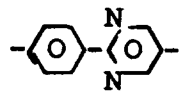 und 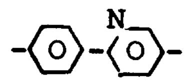 ,

sowie
ferner unsubstituiertes oder ein- oder mehrfach durch Fluor substituiertes 4,4'-Biphenylyl.

$Z^1$ ist bevorzugt eine Einfachbindung, in zweiter Linie bevorzugt -O-CO-, -CO-O-, -C≡C- oder $-CH_2CH_2-$ Gruppen.

Besonders bevorzugt für $Z^1$ ist -CO-O, -O-CO-, -C≡C- oder $-CH_2CH_2-$, insbesondere die $-CH_2CH_2-$ und die -C≡C-Gruppe.

Verbindungen der vor- und nachstehenden Formeln mit verzweigten Flügelgruppen R können von Bedeutung sein. Verzweigte Gruppen dieser Art enthalten in der Regel nicht mehr als zwei Kettenverzweigungen. R ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formel I sind diejenigen bevorzugt, in denen mindestens einer der darin enthaltenen Reste eine der angegebenen bevorzugten Bedeutungen hat.

Diejenigen der vorstehend genannten Formeln, die eine oder mehrere Gruppen Dio, Dit, Pip und/oder Pyr enthalten, umschließen jeweils die beiden möglichen 2,5-(Dio, Dit, Pyr) bzw. 1,4-Stellungsisomeren (Pip).

Bevorzugte Verbindungen der Formel I sind die optisch aktiven Verbindungen der Formel IV

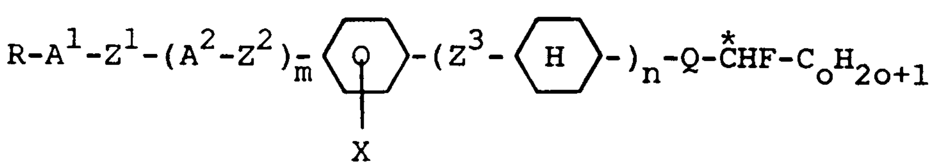 IV

worin

| | |
|---|---|
| R | einen unsubstituierten, einen einfach durch -CN oder einen mindestens einfach durch Fluor oder Chlor substituierten Alkyl- oder Alkenylrest mit bis zu 15 C-Atomen, wobei in diesen Resten auch eine $CH_2$-Gruppe durch -O-, -CO-, -O-CO-, -CO-O- oder -O-CO-O- ersetzt sein kann, |
| $A^1$ und $A^2$ | jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, 1,2,4-Thiadiazol-3,5-diyl-Rest, trans-1,4-Cyclohexylen-Rest, worin auch eine oder zwei nicht benachbarte $CH_2$-Gruppen durch -O- und/oder -S- ersetzt sein können und/oder eine |

CH-Gruppe durch -C(CH)- ersetzt sein kann,

| | |
|---|---|
| $Z^1$, $Z^2$ und $Z^3$ | jeweils unabhängig voneinander -CO-O-, -O-CO-, $-CH_2O-$, $-OCH_2-$, $-CH_2CH_2-$, -CH=CH-, -C≡C- oder eine Einfachbindung, |
| X | H oder F, |
| Q | $-OCH_2$ oder $-CH_2OCH_2-$, |
| o | 1 bis 9, |

und einer der beiden Werte m und n 0 und der andere 0 oder 1 bedeutet, mit den Maßgaben, daß im Falle Q = $-OCH_2$-einer der Ringe $A^1$ und $A^2$ trans-1,4-Cyclohexylen, worin eine CH-Gruppe durch -C(CN)- ersetzt ist, bedeutet,

Insbesondere bevorzugt sind die Verbindungen der Formel IVa bis IVe

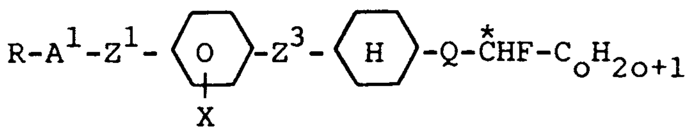

IVa

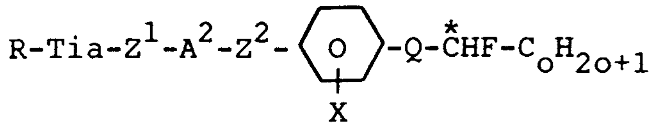

IVb

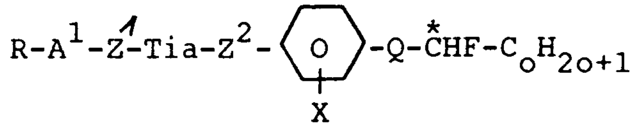

IVc

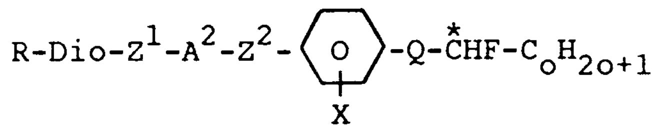

IVd

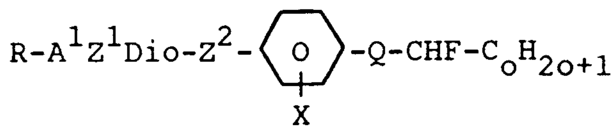

IVe

Weiterhin bevorzugte Verbindungen der Formel I sind chirale oder achirale Ringverbindungen der Formel V

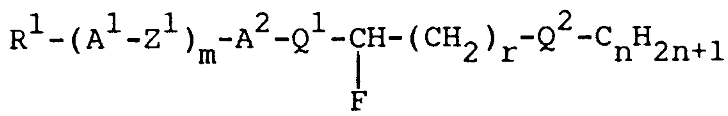

V

worin

| | |
|---|---|
| $R^1$ | eine Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder -CHHalogen- und/oder -CHCN-Gruppen und/oder -O-CO-CHHalogen- und/oder -CO-O-CHCN-Gruppen ersetzt sein können, |
| $A^1$ und $A^2$ | jeweils unabhängig voneinander unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N ersetzt sein können, 1,4-Cyclohexylen, worin auch ein oder zwei nicht benachbarte $CH_2$-Gruppen durch O-Atome und/oder S-Atome ersetzt sein können, Piperidin-1,4-diyl, 1,4-Bicyclo(2,2,2)octylen-, 1,3,4-Thiadiazol-2,5-diyl, Naphthalin-2,6-diyl-, Decahydronaphthalin-2,6-diyl- oder 1,2,3,4-Tetrahydronaphthalin-2,6-diyl-, |
| $Z^1$ | -CO-O-, -O-CO-, $-CH_2CH_2-$, $-OCH_2-$, $-CH_2O-$, -C≡C-oder eine Einfachbindung, |
| m | 1, 2 oder 3, |
| n | 1 bis 7, |

r 1 oder 2,

$Q^1$ $-O-CH_2-$, -O-CO- oder - im Falle $A^1-Z^1-A^2$ =

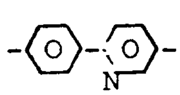, 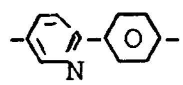, 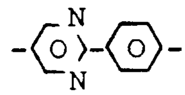 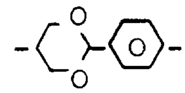 oder

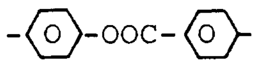

- auch $-CH_2CH_2-$, und

$Q^2$ -O- oder -O-CO- bedeutet.

Insbesondere bevorzugte Verbindungen der Formel V sind diejenige der Formeln V1 bis V6

$$R^1-(A^1-Z^1)_m-A^2-O-CH_2-\underset{\large F}{\underset{|}{CH}}-(CH_2)_r-Q^2-C_nH_{2n+1}$$ V1

$$R^1-(A^1-Z^1)_m-A^2-O-CO_2-\underset{\large F}{\underset{|}{CH}}-(CH_2)_r-Q^2-C_nH_{2n+1}$$ V2

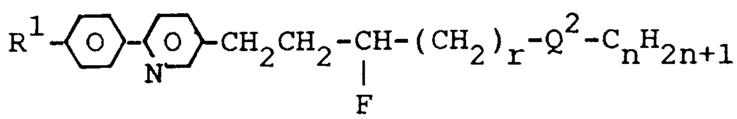 V3

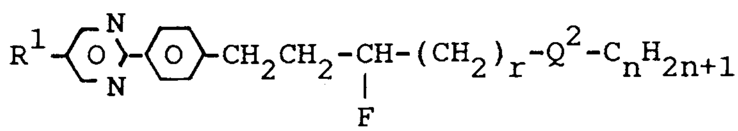 V4

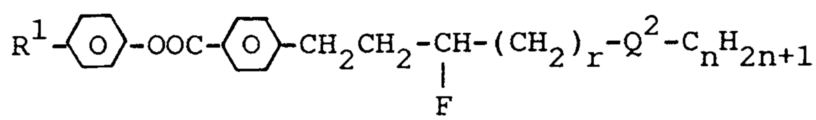 V5

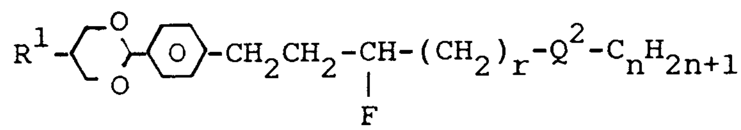 V6

Vor- und nachstehend haben $R^1$, $A^1$, $Z^1$, $A^2$, m, n, $Q^1$ und $Q^2$ die für Formel V angegebene Bedeutung, sofern nicht ausdrücklich etwas anderes vermerkt ist.

Der Rest $-Q^1-CHF-(CH_2)r-Q^2-C_nH_{2n+1}$ wird im Folgenden als $R^*$ bezeichnet.

Die Verbindungen der Formel V umfassen dementsprechend insbesondere Verbindungen der Teilformeln Va (mit zwei Ringen)

$R^1-A^1-Z^1-A^2-R^*$ Va

Vb (mit drei Ringen):

$R^1-(A^1-Z^1)_2-A^2-R^*$ Vb

und Vc (mit vier Ringen):

$R^1-(A^1-Z^1)_3-A^2-R^*$ Vc

Darunter sind diejenigen der Formeln Va und Vb besonders bevorzugt.

Die bevorzugten Verbindungen der Formel Va umfassen solche der Teilformeln Va1 bis Va4:

$R^1$-Ph-$Z^1$-Ph-R* Va1

$R^1$-Ph-$Z^1$-Cy-R* Va2

$R^1$-Cy-$Z^1$-Ph-R* Va3

$R^1$-Cy-$Z^1$-Cy-R* Va4

Darunter sind diejenigen der Teilformeln Va1 besonders bevorzugt.

Die bevorzugten Verbindungen der Formel Vb umfassen solche der Teilformeln Vb1 bis Vb8:

$R^1$-Ph-$Z^1$-Ph-$Z^1$-Ph-R* Vb1

$R^1$-Ph-$Z^1$-Ph-$Z^1$-Cy-R* Vb2

$R^1$-Cy-$Z^1$-Ph-$Z^1$-Cy-R* Vb3

$R^1$-Ph-$Z^1$-Cy-$Z^1$-Ph-R* Vb4

$R^1$-Ph-$Z^1$-Cy-$Z^1$-Cy-R* Vb5

$R^1$-Cy-$Z^1$-Cy-$Z^1$-Ph-R* Vb6

$R^1$-Cy-$Z^1$-Ph-$Z^1$-Cy-R* Vb7

$R^1$-Cy-$Z^1$-Cy-$Z^1$-Cy-R* Vb8

Weiterhin bevorzugte Verbindungen der Formel IV sind
2,5-Disubstituierter Heterocyclus der Formel VI

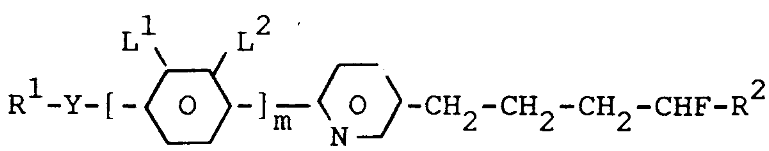

VI

worin

| | |
|---|---|
| $R^1$ | Alkyl, Alkenyl oder Oxaalkyl mit bis zu 12 C-Atomen, |
| $R^2$ | Alkyl mit 2 bis 9 C-Atomen, |
| $L^1$ und $L^2$ | jeweils unabhängig voneinander H oder F, |
| Y | -O-, -CO-O-, -O-CO- oder eine Einfachbindung, und |
| m | 1 oder 2 bedeutet. |

Vor- und nachstehend haben $R^1$, $R^2$, m, $L^1$, $L^2$ und Y die für Formel VI angegebene Bedeutung, sofern nicht ausdrücklich etwas anderes vermerkt ist.

Die Verbindungen der Formel VI umfassen dementsprechend insbesondere Verbindungen der Teilformeln VIa und VIb:

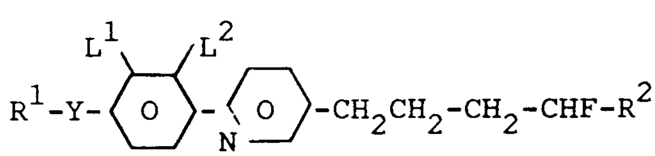

VIa

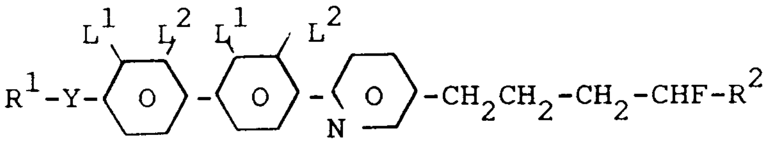 VIb

Darunter sind diejenigen der Formel VIa besonders bevorzugt.

Y ist vorzugsweise -O- oder -CO-O-, insbesondere bevorzugt -O-.

m ist vorzugsweise 1.

Der Rest

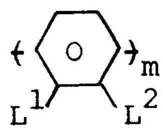

hat vorzugsweise eine der nachfolgenden Bedeutungen 1 bis 6:

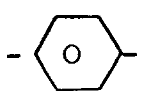

1

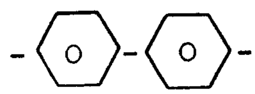

2

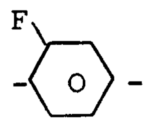

3

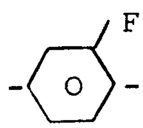

4

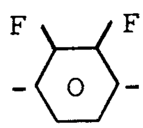

5

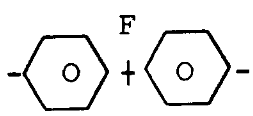

6

Die Bedeutungen 1 und 2, insbesondere 1, sind besonders bevorzugt. Die Position des Fluors in 6 ist beliebig.

Die Verbindungen der Formel VI sind vorzugsweise optisch aktiv und werden als chirale Dotierstoffe für ferroelektrische Mischungen verwendet. -CHF- ist vorzugsweise ein asymmetrisches C-Atom.

$R^2$ ist vorzugsweise geradkettiges oder verzweigtes Alkyl mit bis zu 10 C-Atomen, vorzugsweise mit 3 bis 8 C-Atomen.

Weiterhin bevorzugte Verbindungen der Formel I sind chirale oder achirale Ringverbindungen der Formel VII

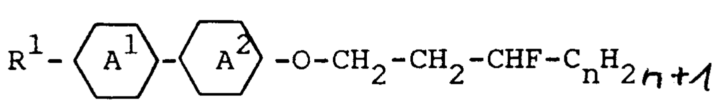 VII

worin

- $R^1$ eine Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder -CHHalogen- und/oder -CHCN-Gruppen und/oder -O-CO-CHHalogen- und/oder -CO-O-CHCN-Gruppen ersetzt sein können
- $A^1$ und $A^2$ jeweils unabhängig voneinander unsubstituiertes oder durch ein oder zwei F-Atome substituiertes 1,4-Phenylen, worin auch eine oder zwei CH-Gruppen durch N ersetzt sein

können,

n 1 bis 9 bedeutet.

Vor- und nachstehend haben $R^1$, $A^1$, $A^2$ und n die für Formel VII angegebene Bedeutung, sofern nicht ausdrücklich etwas anderes vermerkt ist.

Der Rest $-O-CH_2-CH_2-CHF-C_nH_{2n+1}$ wird im folgenden als R* bezeichnet.

Die Verbindungen der Formel VII umfassen dementsprechend insbesondere die bevorzugten Verbindungen der Teilformeln VIIa bis VIIf:

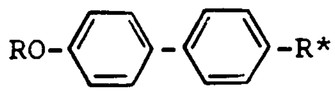

VIIa

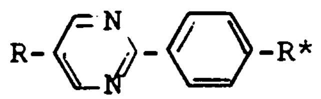

VIIb

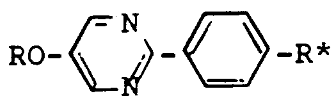

VIIc

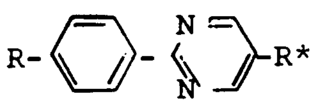

VIId

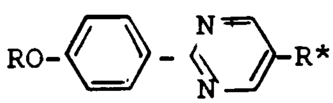

VIIe

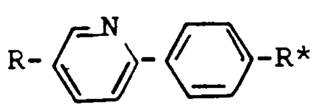

VIIf

Darunter sind diejenigen der Formeln VIIb, VIIc und VIIe besonders bevorzugt.

$A^1$ und $A^2$ sind bevorzugt jeweils unabhängig voneinander eine 1,4-Phenylen- (Phe), eine Pyrimidin-2,5-diyl-(Pyr), eine Pyridin-2,5-diyl- (Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind.

Besonders bevorzugt sind Verbindungen der Formel VII und der vorstehenden Teilformeln, die eine Gruppierung -Phe-Phe-, Phe-Pyr oder Phe-Pyn enthalten. Besonders bevorzugt sind die Gruppen

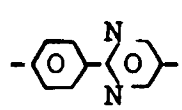 und 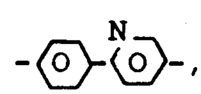,

wobei die 1,4-Phenylenringe auch durch ein oder zwei Fluor-Atome substituiert sein können, sowie ferner unsubstituiertes oder ein- oder mehrfach durch Fluor substituiertes 4,4'-Biphenylyl.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die optisch aktiven Verbindungen der Formel I erhält man durch den Einsatz entsprechender optisch aktiver Ausgangsmaterialien und/oder durch Trennung der optischen Antipoden mittels Chromatographie nach bekannten Methoden.

So können zur Herstellung von Verbindungen der Formel V mit r = 2 geeignete Vorstufen aus optisch aktiver Äpfelsäure nach folgendem Reaktionsschema (ta/1-3) hergestellt werden:

Schema 1

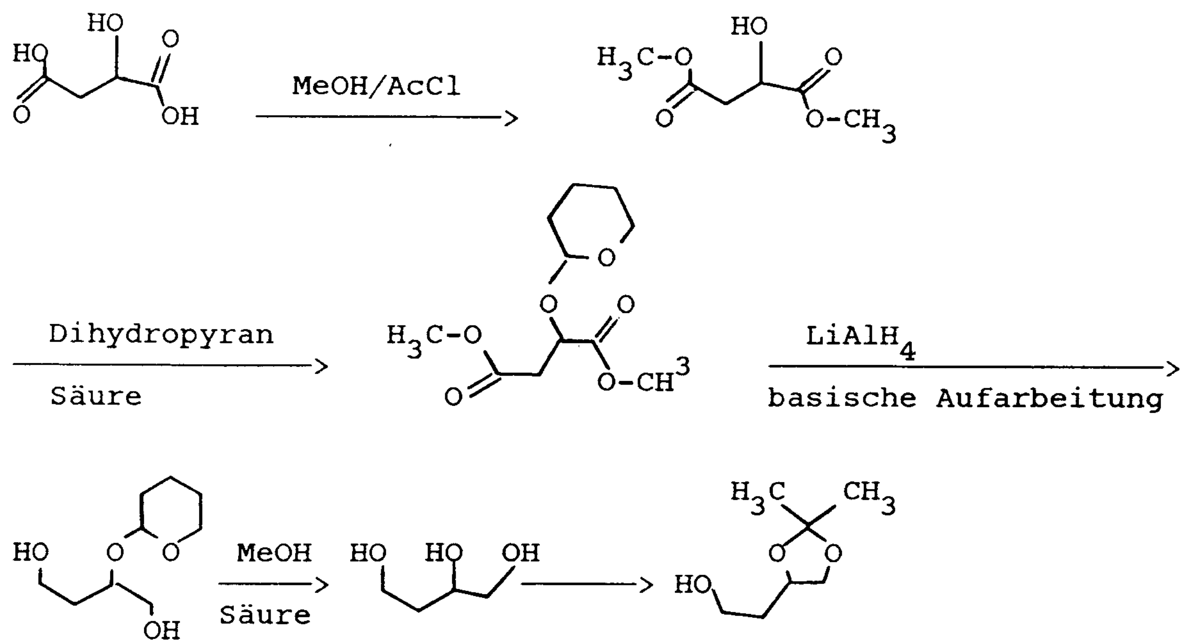


Bis zu dieser Stufe ist die Synthese von Mori et al. beschrieben worden (K. Mori, T. Takigawa and T. Matsuo, Tetrahedron 35, 933-944 (1979).

Später haben dann Meyers and Lawsson gefunden, daß die chemische Reinheit des auf diesem Weg erhaltenen Acetonids nur etwa 90 % beträgt (A.I. Meyers and J.P. Lawson, THL 23, 4883-4886 (1982).

Dessen ungeachtet kann die freie Alkoholgruppe des Acetonids nach einer der üblichen Methoden verethert werden (z.B. C.A. Brown and D. Barton, Synthesis (1974) 434 oder B.R. Jursic, Tetrahedron 44, 6677-6680 (1988).

Der Benzylether (K. Isaac and P. Kocienski, J. Chem. Soc., Chem. Commun. (1982) 460-462) bietet sich insbesondere als Schutzgruppe an, da er später leicht hydrogenolytisch abgespalten werden kann. Unter Standardbedingungen wird nach der Veretherung das Isopropylidenketal zum 1,2-Diol hydrolisiert und dieses dann entsprechend den Reaktionsbedingungen von Di Fabio und Misiti in das entsprechende Epoxid überführt (R. Di Fabio and D. Misiti, Gazetta Chimica Italiana 118, 209-210 (1988)).

Die Behandlung des Acetonids mit HBr/Eisessig und die anschließende Umsetzung der auf diese Weise erhaltenen Bromooxalkylacetate mit K-Pentanolat liefert entsprechend der Arbeit von U. Schmidt et al. ebenfalls die gewünschten Epoxide (U. Schmidt, J. Tabiersky, F. Bartowiak and J. Wild, Angew. Chem. 92, 201-202 (1980)).

Schema 2

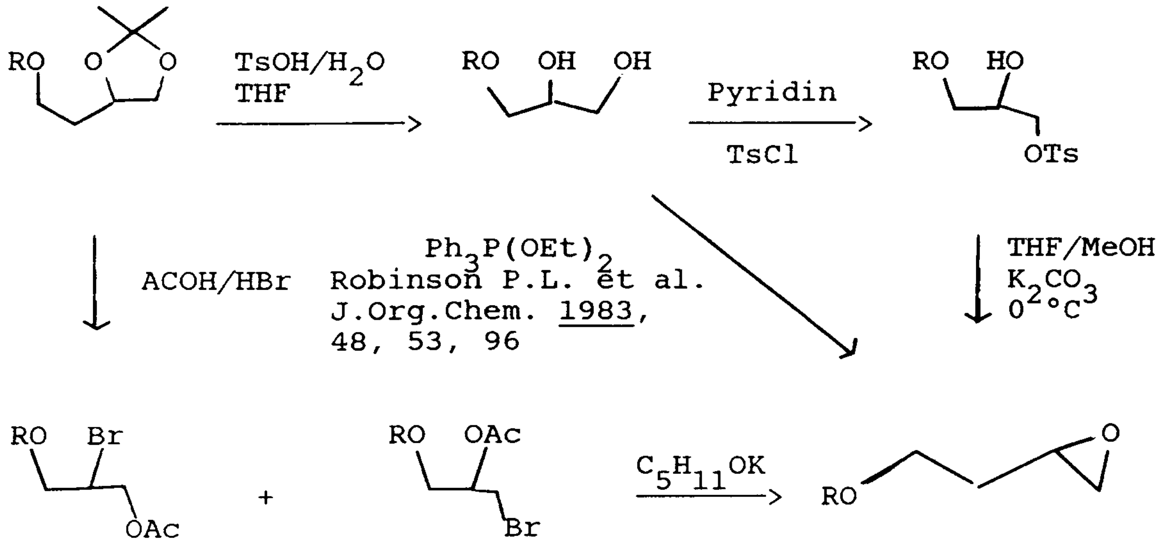


Öffnet man das Epoxid mit Pyridin/HF (N. Mongelli, F. Animati et al., Synthesis 310 (1988)), dann erhält man den entsprechenden Fluoralkohol, der anschließend in das entsprechende Tosylat überführt werden kann. Solche Tosylate eignen sich insbesondere zur Alkylierung von Phenolen.

Schema 3

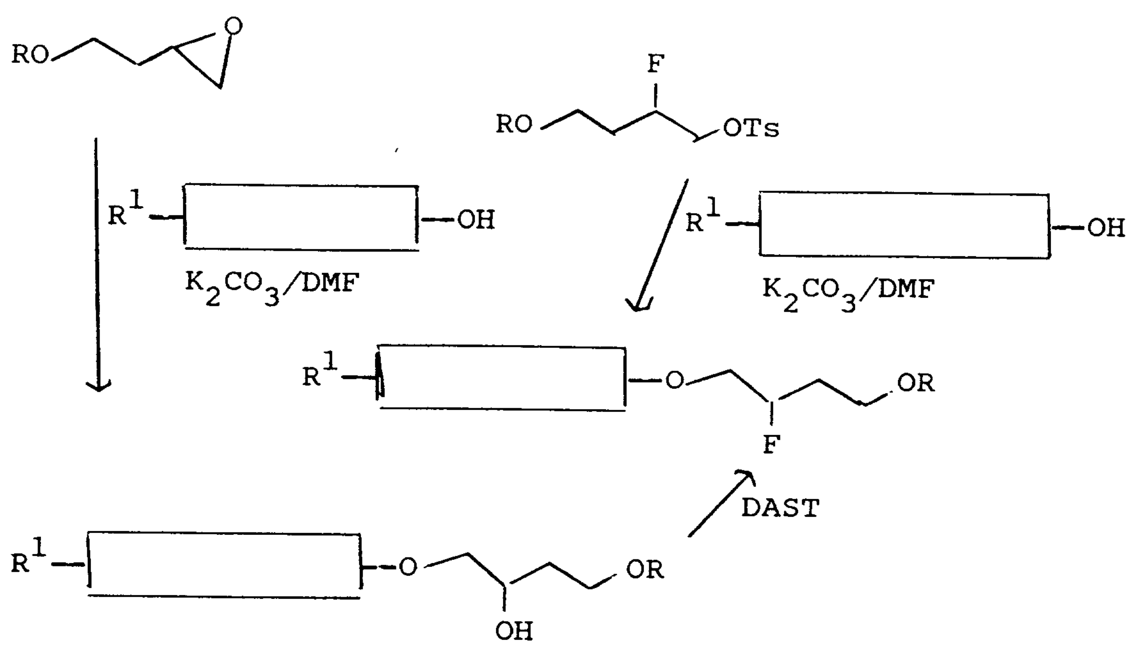


Wie obiges Reaktionsschema zeigt, kann man das Epoxid auch direkt mit Phenolen umsetzen. Das Epoxid wird mit hoher Selektivität am weniger substituierten Kohlenstoffatom zum chiralen sekundären Alkohol geöffnet, der dann schließlich mit DAST unter Inversion in die erfindungsgemäßen Verbindungen überführt wird. Zu den üblichen Umsetzungen von Alkoholen mit DAST siehe: M. Hudlicky, Organic Reactions 35 513-637 (1987).

Die erfindungsgemäßen Verbindungen der Formel V mit $Q^2$ = -O-CO- lassen sich aus den entsprechenden Benzyethern durch Hydrogenolyse und anschließende Veresterung herstellen. Folgendes Synthe-

seschema beschreibt die Herstellung:

Schema 4

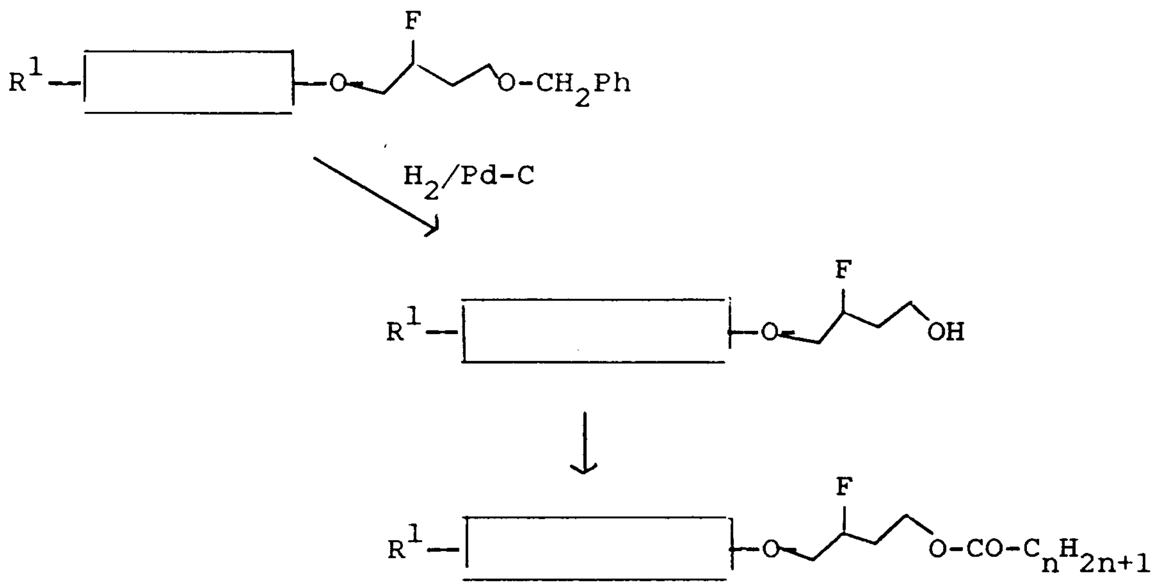


Weiterhin erhält man die Verbindungen mit $Q^1$ = -O-CO-durch Oxidation der entsprechenden Fluoralkohole und anschließende Veresterung mit mesogenen Phenolen:

Schema 5

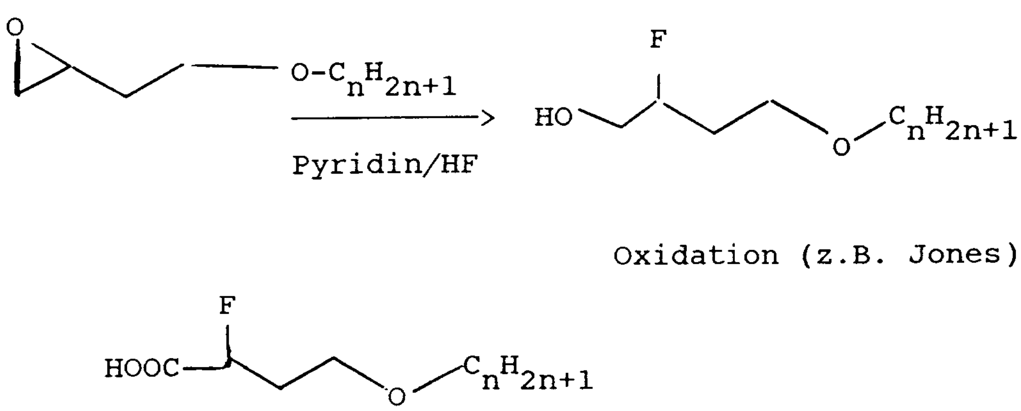


Wenn bei der Oxidation Racemisierung auftritt, kann man die optisch aktiven Fluorsäuren durch Racematspaltung nach Helmchen gewinnen (Angew. Chem. 91, 65 (1979)). CH-azide Verbindungen, wie beispielsweise Tolunitril oder Methylpyridine, öffnen in Gegenwart geeigneter Basen ebenfalls das Epoxid zum optisch aktiven sekundären Alkohol, der dann mit DAST unter Inversion fluoriert wird. Bevorzugt Reaktionswege können der folgenden Schemata (6-9) entnommen werden.

Schema 6

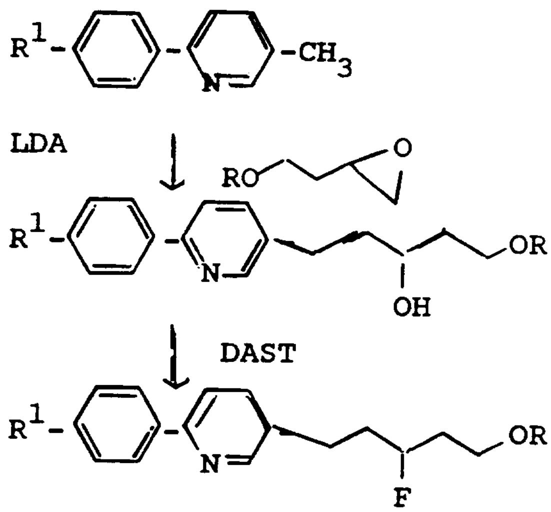


Schema 7

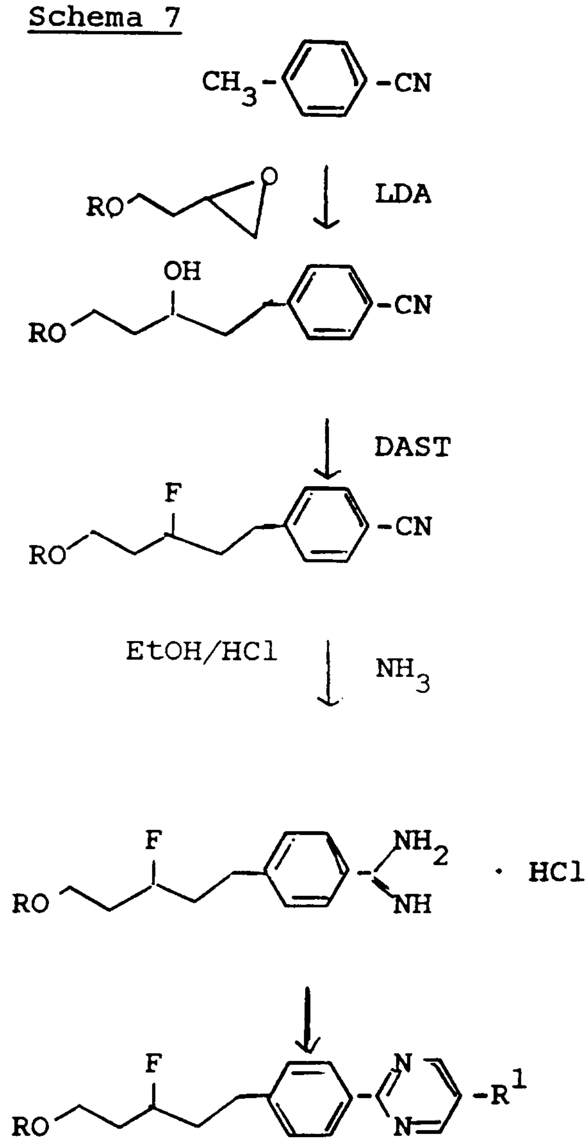

Aus den Nitrilen kann man durch milde Verseifung über die Iminoether nach Pinner die entsprechenden Benzoesäuren erhalten:

Schema 8

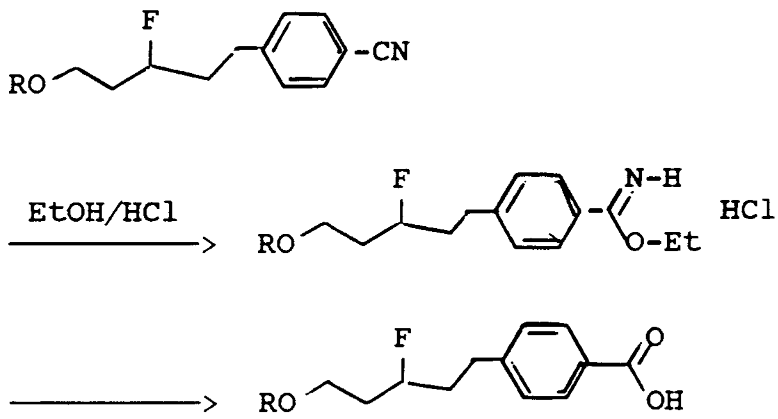


Diese Säuren können mit Hydroxygruppen typischer Flüssigkristallbausteine verestert werden.

Die Verbindungen der Formel I mit r = 1 können analog hergestellt werden unter Einsatz der bekannten Epoxide der Formel

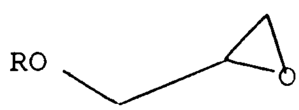

oder der aus diesen nach üblichen Verfahren erhältlichen Fluoralkohole der Formel

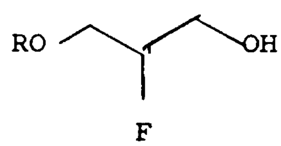

Die Synthese der bevorzugten Phenylpyridine ist im Folgenden näher angegeben:

Schema 9

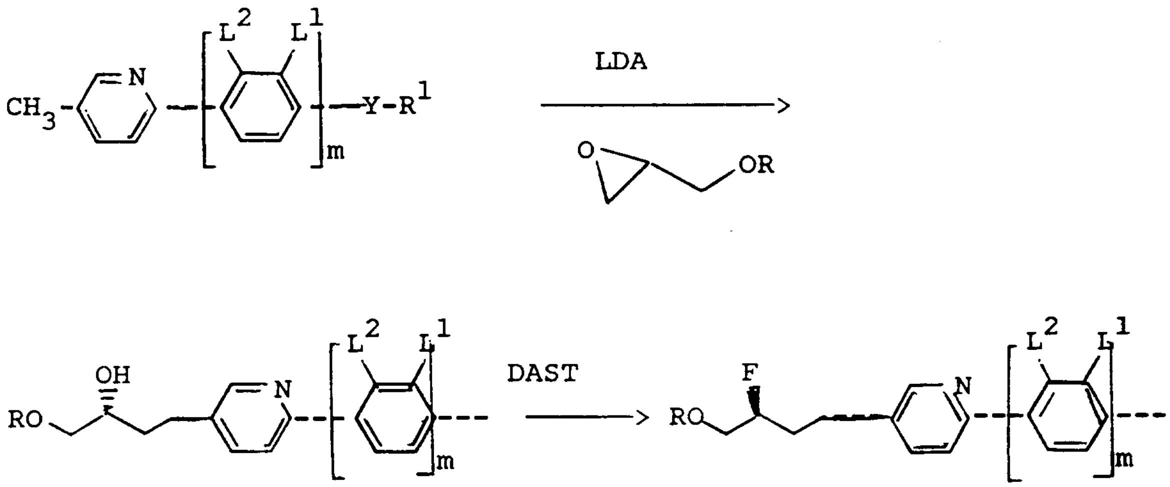


In den vorstehenden Formeln bedeutet R jeweils $C_nH_{2n+1}$ (n = 1 bis 7) und

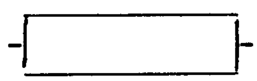

bedeutet $-(A^1-Z^1)_mA^2-$.

So können die Verbindungen der Formel VI oder zu deren Herstellung geeignete Vorstufen hergestellt werden, indem man eine Verbindung der Formel VI',

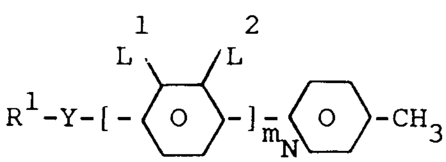

VI'

worin $R^1$, Y, $L^1$, $L^2$ und m die oben angegebene Bedeutung haben, oder eine geeignete Vorstufe unter basischen Bedingungen mit einer Verbindung der Formel VI'' umsetzt,

$R^2$-CHF-$CH_2$-$CH_2$-Hal VI''

worin $R^2$ die oben angegebene Bedeutung hat und Hal vorzugsweise Br oder I bedeutet.

Zur Herstellung von Verbindungen der Formel VI'' können geeignete Vorstufen nach Schema 1 und 2 aus optisch aktiver Äpfelsäure hergestelltem Epoxid nach folgendem Reaktionsschema (10) hergestellt werden:

Schema 10

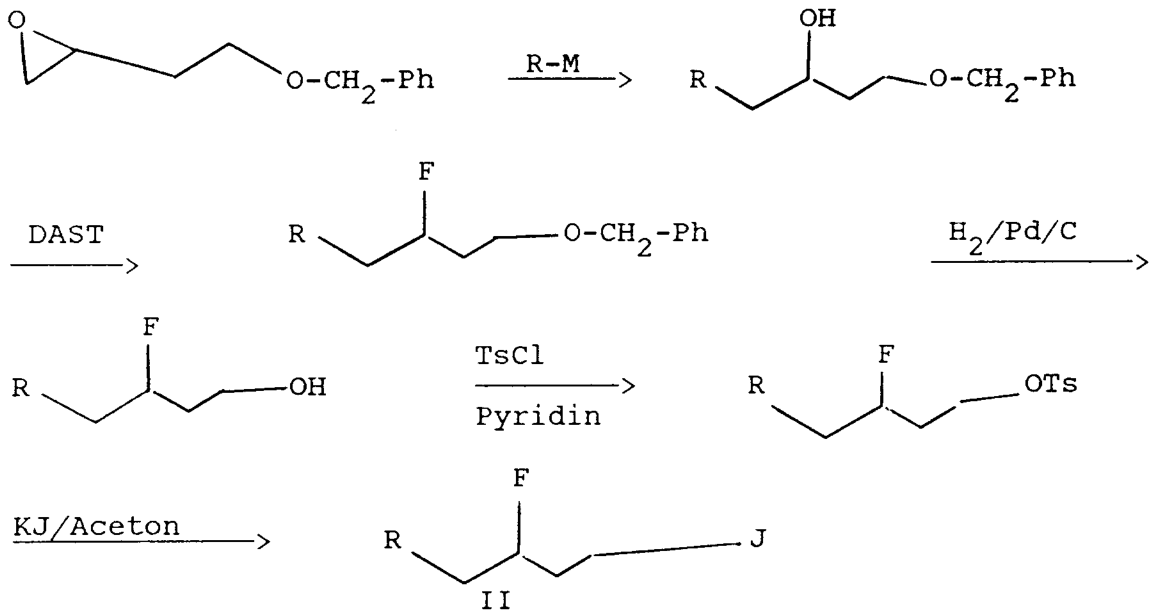


II

Die Umsetzung des Epoxids mit metallorganischen Verbindungen, bevorzugt mit Grignard-Verbindungen, ergibt unter Ringöffnung am weniger substituierten C-Atom des Epoxids mit hoher Selektivität den ensprechenden Alkohol, der mit DAST unter Standardbedingungen fluoriert wird. Hydrogenolyse liefert schließlich den primären Alkohol, dessen reaktive Derivate VI'' nach Standardmethoden zugänglich sind.

Die Reaktionsbedingungen der Umsetzung von VI' mit VI'' sind an sich nicht kritisch. Man metalliert die 2-substituierten 5-Methylpyridine der Formel VI' unter den in DE 36 32 411 (Beispiel 3) genannten Bedingungen (wobei auf die Verwendung von DMPU verzichtet werden kann) und gibt dann bei -10 °C eine äquimolare Menge ggf. chirales Halogenid der Formel VI'' zu.

Die Ausgangsmaterialien der Formel VI' sind z.B. erhältlich aus 2-p-Methoxyphenyl-5-methyl pyridin durch basische Etherspaltung mit K-tert.-butylat in N-Methylpyrrolidon (NMP) bei 150° - 200°C und anschließende erneute Veretherung mit den entsprechenden Alkylhalogeniden oder durch Kreuzkopplung der entsprechenden aromatischen Boronsäuren mit 2-Brom-5-methylpyridin nach M.J. Sharp, W. Cheng und V. Snieckus, Tetrahedron Letters 28, 5093 (1987).

Die chiralen Verbindungen der Formel I, worin $Q^1$ -O-$CH_2$- oder $CH_2CH_2$ und $Q^2$ $(CH_2)_3$-O- bedeuten, sind besonders bevorzugt. Sie lassen sich gemäß Schema 11 aus chiralen Epoxiden des Typs

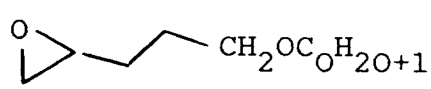

durch Ringöffnung mit Phenolaten oder CH-aziden Verbindungen herstellen. Das entsprechende 4-Hydroxymethyl-γ-butyro - lacton ist beschrieben (Il Farmaco 44 (3), 303-313, 1989).

Schema 11

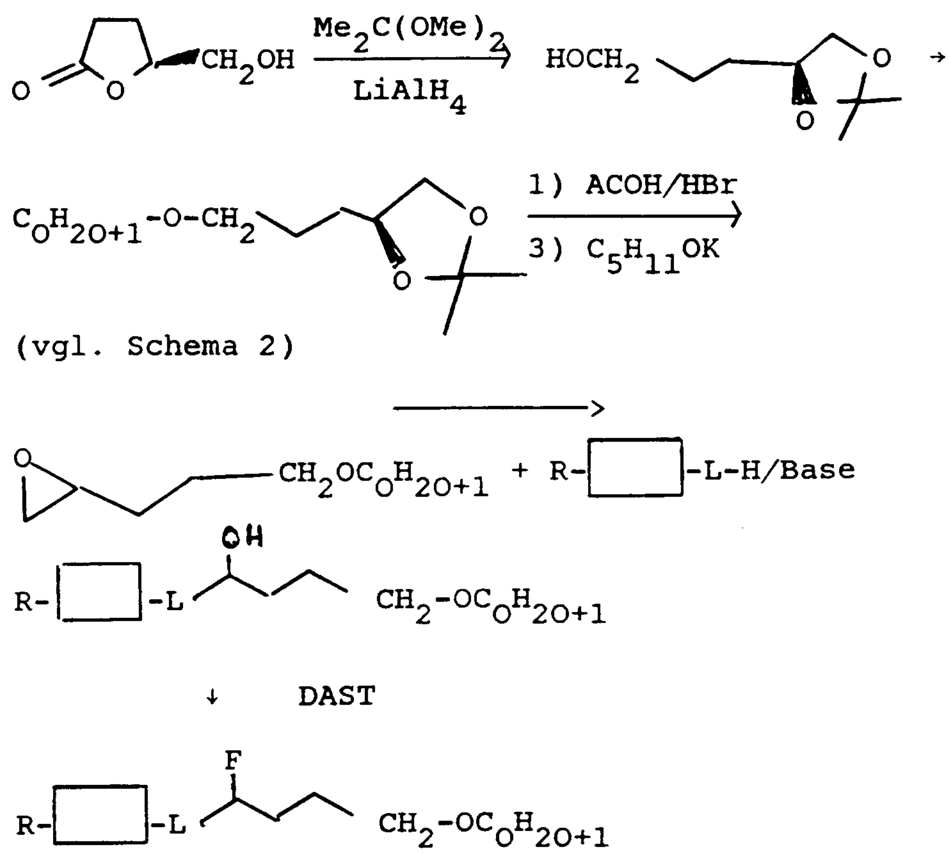


(vgl. Schema 2)

Dabei bedeuten:

L O oder $CH_2$, und

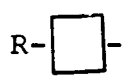

$R\text{-}A^1\text{-}Z^1\text{-}(A^2\text{-}Z^2)_m\text{-}(Z^3\text{-} H \text{-})_n$

Die erfindungsgemäßen Phasen enthalten mindestens eine, vorzugsweise mindestens zwei Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Die Chiralität beruht vorzugsweise teilweise oder vollständig auf chiralen Verbindungen der Formel I. Diese Phasen enthalten vorzugseise eine oder zwei chirale Verbindungen der Formel I. Es können jedoch auch achirale Verbindungen der Formel I (zum Beispiel in Form eines Racemates) eingesetzt werden, wobei dann die Chiralität der Phase durch andere optisch aktive Verbindungen hervorgerufen wird. Falls chirale Verbindungen der Formel I zum Einsatz kommen, eignen sich neben den reinen optischen Antipoden auch Gemische mit einem Enantiomerenüberschuß. Die oben erwähnten weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln VIIIa bis VIIIp:

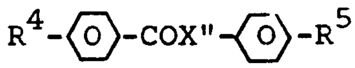
VIIIa

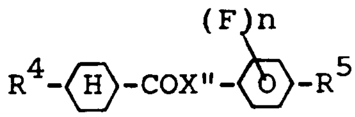
VIIIb

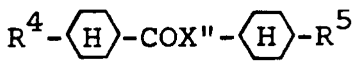
VIIIc

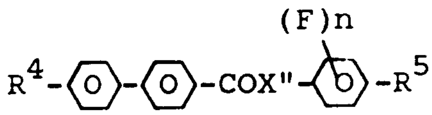
VIIId

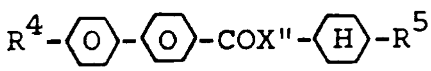
VIIIe

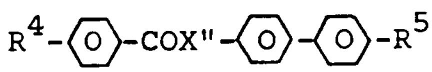
VIIIf

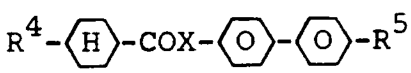
VIIIg

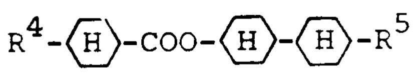
VIIIh

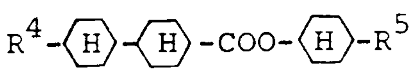
VIIIi

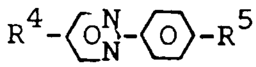
VIIIj

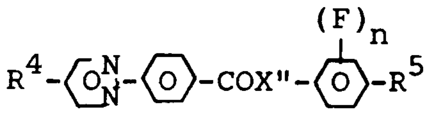
VIIIk

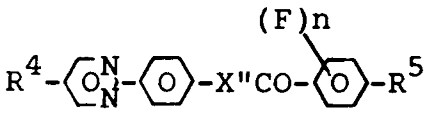
VIIIl

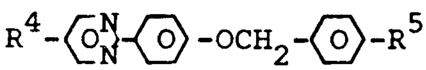
VIIIm

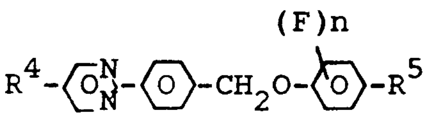
VIIIn

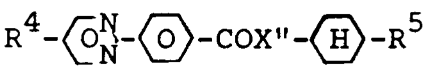
VIIIo

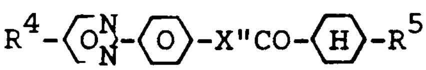
VIIIp

$R^4$ und $R^5$ sind jeweils vorzugsweise geradkettiges oder verzweigtes Alkyl, Alkoxy, Alkanoyloxy oder Alkoxycarbonyl mit jeweils 3 bis 12 C-Atomen. X" ist O oder S, vorzugsweise 0. n ist 0 oder 1.

Besonders bevorzugt sind die Verbindungen der Teilformeln VIIIa, VIIIb, VIIId und VIIIf, worin $R^4$ und $R^5$ jeweils geradkettiges Alkyl oder Alkoxy mit jeweils 5 bis 10 C-Atomen bedeutet.

Die Verbindungen der Teilformeln VIIIc, VIIIh und VIIIi eignen sich als Zusätze zur Schmelzpunkterniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln VIIIc, VIIIh und VIIIi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

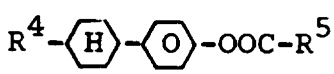

worin $R^4$ und $R^5$ die für VIIIc, VIIIh und VIIIi angegebene bevorzugte Bedeutung haben.

Als weitere Komponenten mit negativer dielektrischer Anisotropie eignen sich weiterhin Verbindungen enthaltend das Strukturelement M, N-oder O.

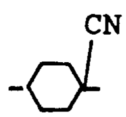
M

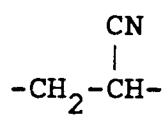
N

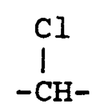
O

Bevorzugte Verbindungen dieser Art entsprechen den Formeln IXb und IXc:

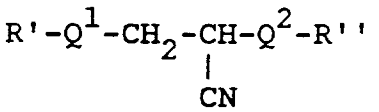 IXb

R'-$Q^3$-$Q^4$-R''' IXc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

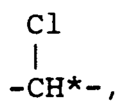, 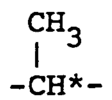 oder 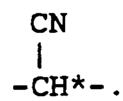.

R''' hat vorzugsweise die Formel

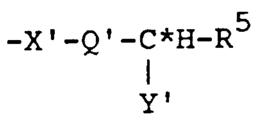

mit den vorgehend genannten bevorzugten Bedeutungen.

Besonders bevorzugte Komponenten mit negativer dielektrischer Anisotropie sind die in der WO 86-00529 beschriebenen Verbindungen mit dem Strukturelement M oder N. Besonders bevorzugt sind diejenigen der Formel IXd

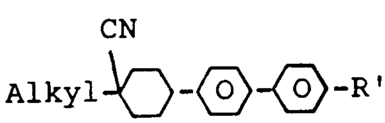 IXd

worin Alkyl eine geradkettige oder verzweigte Alkylgruppe mit vorzugsweise 3 bis 10 C-Atomen ist und R' die oben angegebene Bedeutung hat. Ferner bevorzugt sind Verbindungen entsprechend der Formel IXd, worin eine oder beide die Ringe verknüpfenden Einfachbindungen durch eine Gruppe ausgewählt aus $-CH_2CH_2-$, -O-CO- oder -CO-O- ersetzt sind. Besonders bevorzugte Verbindungen der Formel VIc sind diejenigen der Formel IXc':

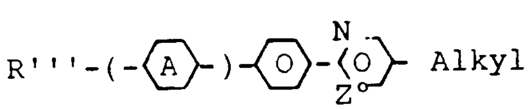

worin A 1,4-Phenylen oder trans-1,4-Cyclohexylen, Z° CH oder N und n 0 oder 1 bedeutet.

A trans-1,4-Cyclohexylen, 1,4-Phenylen oder 1,3-Cyclobutylen,

Q $-CH_2-$ oder -CO-,

r 0 oder 1, und

n 1 bis 9 bedeutet.

Die neuen Verbindungen der Formeln II und III besitzen einen breiten Anwendungsbereich In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formeln II und III flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formeln II und III eignen sich ferner als Zwischenprodukte zur Herstellung anderer Substanzen, die sich als Bestandteile flüssigkristalliner Phasen verwenden lassen.

Die Verbindungen der Formeln II und III sind in reinem Zustand farblos und weisen günstige Werte der optischen Anisotropie auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel II und III als Komponenten chiraler getilteter smektischer Phasen vorteilhaft eingesetzt werden. Chemisch, thermisch und gegen Licht sind sie sehr stabil.

Gegenstand der Erfindung sind somit die Verbindungen der Formeln II und III sowie die Verwendung der Verbindungen der Formeln II und III als Komponenten flüssigkristalliner Phasen.

Gegenstand der Erfindung sind auch chirale getiltete smektische flüssigkristalline Phasen mit einem Gehalt an mindestens einer Verbindung der Formeln II und III.

Gegenstand der Erfindung sind ferner elektrooptische Anzeigeelemente, die derartige Phasen enthalten.

Gegenstand der Erfindung sind schließlich auch neue Zwischenprodukte der Formel II'

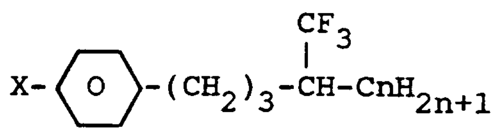

II'

worin

n 1 bis 9,

$R^2$ Alkyl, Alkenyl oder Oxaalkyl mit bis zu 12 C-Atomen, und

X Cyan, Carboxy, Amidin oder Hydroxymethyl bedeutet,

sowie deren reaktionsfähige Derivate.

Vor- und nachstehend haben R, A, Q, r und n die für die Verbindungen der Formeln II und III angegebene Bedeutung, sofern nicht ausdrücklich etwas anderes vermerkt ist.

Verbindungen der vor- und nachstehenden Formeln mit verzweigten Flügelgruppen R können von Bedeutung sein. Verzweigte Gruppen dieser Art enthalten in der Regel nicht mehr als zwei Kettenverzweigungen. R ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oza-4-methylpentyl.

Der Rest R kann auch ein optisch aktiver organischer Rest mit einem asymmetrischen Kohlenstoffatom sein.

R bedeutet vorzugsweise Alkyl oder Alkenyl mit bis zu 12 C-Atomen. Besonders bevorzugt sind Alkyl mit 2 bis 12 C-Atomen, d.h. Ethyl, Propyl, Butyl, Pentyl, Hexyl, Heptyl, Octyl, Nonyl, Decyl, Undecyl und Dodecyl. Diese Gruppen können geradkettig oder verzweigt sein, wobei geradkettige Alkylgruppen bevorzugt sind. R bedeutet jedoch auch bevorzugt Methyl oder verzweigtes Alkyl mit einer Methylverzweigung, z.B. iso-Propyl.

r ist vorzugsweise O.

Die bevorzugten Verbindungen, worin R Alkyl mit bis zu 9 C-Atomen bedeutet, sind vorzugsweise optisch aktiv und werden als chirale Dotierstoffe für ferroelektrische Mischungen verwendet. Das Racemat selbst kann als Basismaterial für derartige Mischungen verwendet werden.

Unter den Verbindungen der Formeln II und III I sind diejenigen bevorzugt, in denen mindestens einer der darin enthaltenen Reste eine der angegebenen bevorzugten Bedeutungen hat.

Die Verbindungen der Formeln II und III werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formeln II und III umsetzt.

So können die Verbindungen der Formel II oder zu deren Herstellung geeignete Vorstufen hergestellt werden, indem man p-Tolunitril unter basischen Bedingungen mit 3-Trifluormethylalkyljodiden umsetzt, die ihrerseits durch Finkelstein-Reaktion (NaJ/Aceton) aus den aus EP-A-0301511 bekannten Tosylaten erhältlich sind. Vorzugsweise wird ein optisch aktives 3-Trifluormethylalkyljodid verwendet.

Die Reaktionsbedingungen der Umsetzung von Tolunitril mit den Trifluormethylalkyliodiden sind an sich nicht kritisch. Man metalliert unter den in DE 36 32 411 (Beispiel 3) genannten Bedingungen (wobei auf die Verwendung von DMPU ggf. verzichtet werden kann) und gibt dann bei -10 °C eine äquimolare Menge ggf. chirales Trifluormethylalkyljodid zu.

Man erhält die erfindungsgemäßen Fluorverbindungen der Formel II' (X = CN), aus welchem man durch Behandeln mit EtOH/HCl und $NH_3$ das entsprechende Amidinhydrochlorid erhält.

Die weitere Synthese der erfindungsgemäßen Verbindungen erfolgt gemäß folgendem Schema:

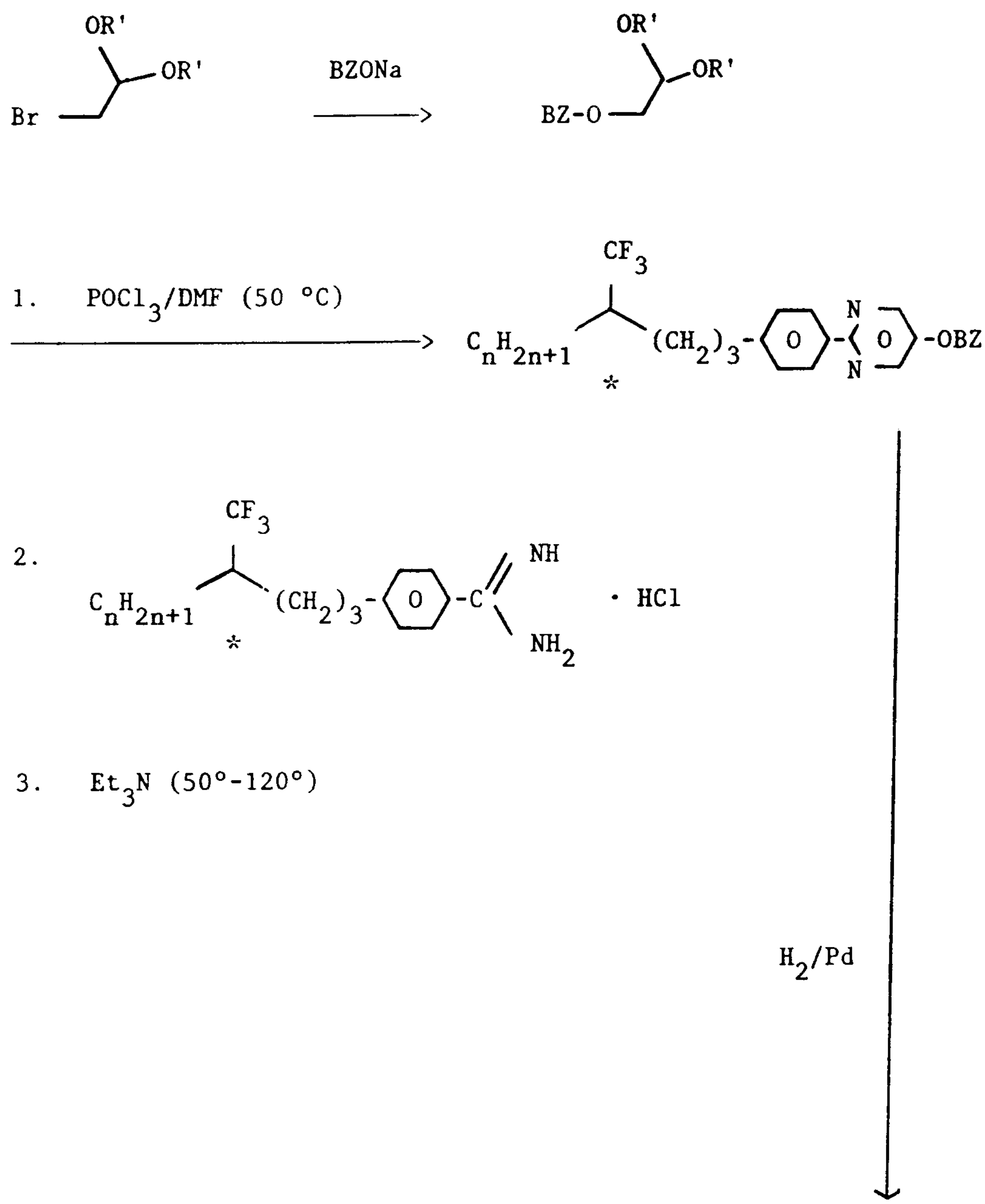

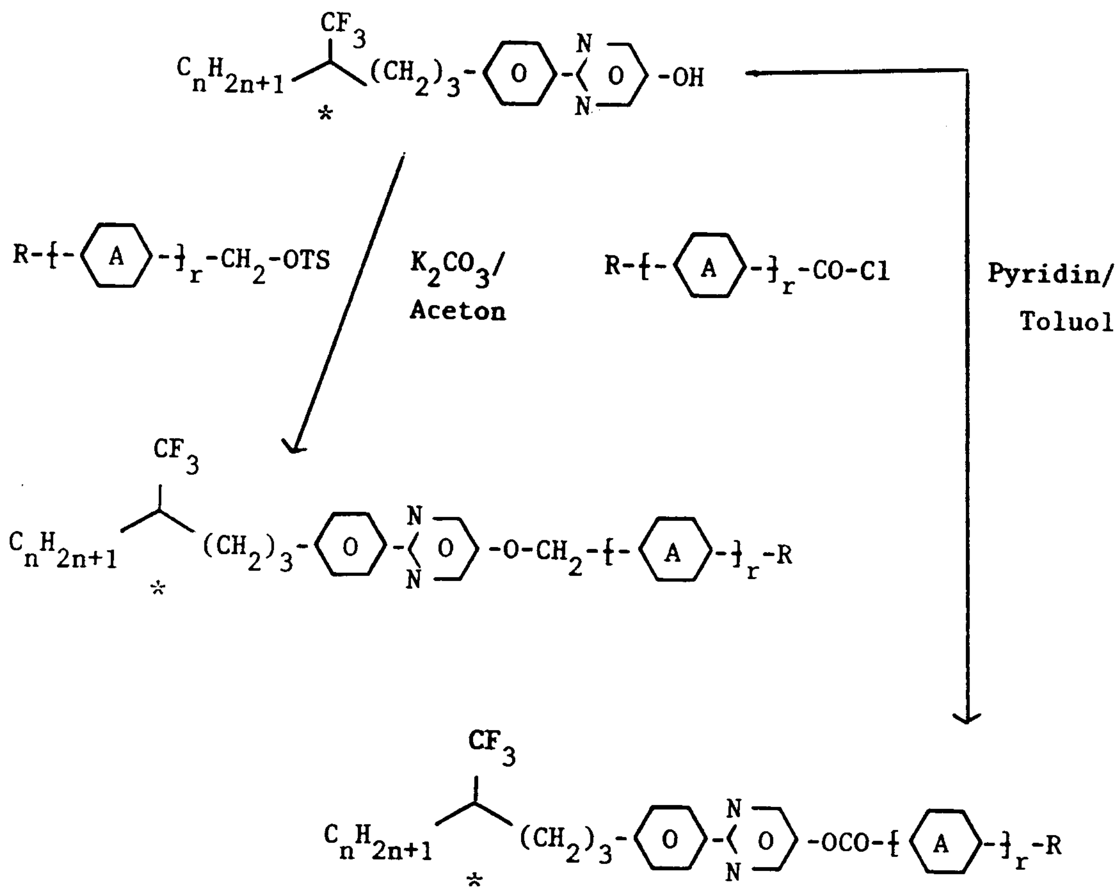


So können die Verbindungen der Formel III oder zu deren Herstellung geeignete Vorstufen hergestellt werden, indem man eine Verbindung der Formel III',

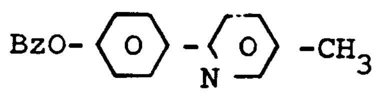

III'

unter basischen Bedingungen mit einem Trifluormethylalkyljodid der Formel III"

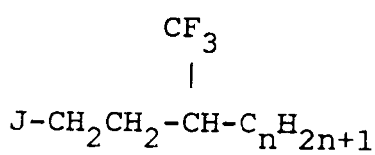

III"

umsetzt, worin n 1 bis 9 bedeutet. Vorzugsweise wird eine optisch aktive Verbindung II verwendet.

Die Reaktionsbedingungen der Umsetzung von III' mit III" sind an sich nicht kritisch. Man metalliert die 2-substituierten 5-Methylpyridine der Formel I' unter den in DE 36 32 411 (Beispiel 3) genannten Bedingungen (wobei auf die Verwendung von ggf. DMPU verzichtet werden kann) und gibt dann bei -10 °C eine äquimolare Menge ggf. chirales III" zu.

Nähere Einzelheiten können dem folgenden Schema entnommen werden:

Schema

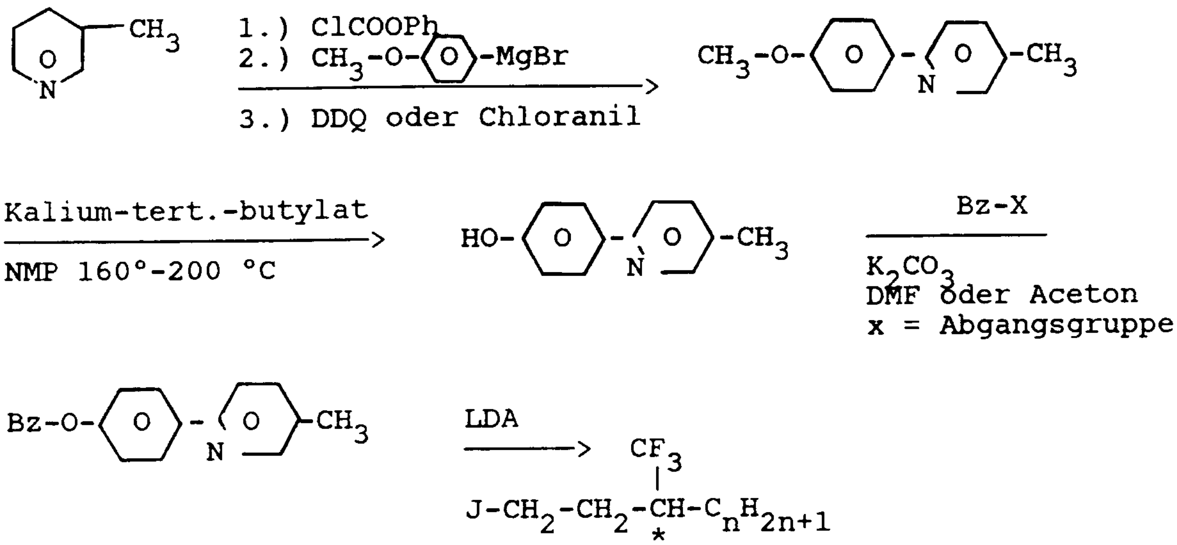


Die Verbindungen der Formel I, eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I, II und/oder III. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel X charakterisieren,

R'-L-G-E-R''     X

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G -CH=CH- -N(O)=N-
-CH=CY- -CH=N(O)-
-C≡C- $-CH_2-CH_2-$
-CO-O- $-CH_2-O-$
-CO-S- $-CH_2-S-$
-CH=N- -COO-Phe-COO-
oder eine C-C-Einfachbindung,

Y Halogen, vorzugsweise Chlor, oder -CN, und

R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $-OCF_3$, $-OCF_2H$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249-258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

0,05 mol trans-4-(4'-Octyloxybiphenyl-4-yl)-cyclohexancarbonsäurechlorid(hergestellt aus 4-Octyloxy-4'-brombiphenyl durch Lithiierung, Überführung in eine titanorganische Verbindung und Reaktion mit 4-Cyclohexanoncarbonsäureethylester, Dehydratisierung und Hydrierung der Doppelbindung, Verseifung des Esters und Umsetzung der Säure mit Thionylchlorid), 0,05 mol (S)-2-Fluoroctanol und 0,05 mol Pyridin werden in 100 ml Toluol 5 Stunden zum Rückfluß erhitzt. Nach dem Abkühlen saugt man das Pyridinhydrochlorid ab und engt das Filtrat zum Rückstand ein.

Das reine (S)-2-Fluoroctyl-[trans-4-(4'-octyloxybiphenyl-4-yl)-cyclohexan]-carboxylat wird durch Kristallisation aus Ethanol erhalten.

Beispiel 2

0,1 mol 4-Hydroxybenzoesäuremethylester, 0,1 mol (S)-2-Fluoroctanol-1 und 0,12 mol Triphenylphosphin werden in 250 ml Tetrahydrofuran gelöst und unter Rühren und Eiskühlung 0,12 mol Diethylazodicarboxylat zugetropft. Man läßt auf Raumtemperatur kommen und rührt noch 8 Stunden nach. Danach wird das Lösungsmittel destilliert und das Methyl-4-(2-fluor-octyloxy)-benzoat durch Säulenchromatographie gereinigt. Durch Verseifung mit wäßrig/alkoholischer Kalilauge erhält man daraus die (S)-4-(2-Fluoroctyloxy)-benzoesäure.

0,01 mol dieser Säure, 0,001 mol 4-Dimethylaminopyrimidin und 0,01 mol 4-n-Octylphenol werden in 15 ml Dichlormethan vorgelegt, bei 10° unter Rühren eine Lösung von 0,01 mol Dicyclohexylcarbodiimid zugetropft und anschließend 15 Stunden bei Raumtemperatur nachgerührt. Man filtriert über Kieselgel ab, verdampft das Lösungsmittel und erhält als Rückstand (S)-(4-n-Octylphenyl)-4-(2-fluoroctyloxy)-benzoat.

Beispiel 3

Analog erhält man aus (S)-4-(2-Fluoroctyloxy)-benzoesäure und 2-(4-Hydroxyphenyl)-5-n-nonylpyrimidin (S)-{4-(5-n-Nonylpyrimidin-2-yl)-phenyl}-4-(2-fluoroctyloxy)-benzoat.

Beispiel 4

4-{5-[2-(4-Pentylphenyl)-ethinyl]-pyrimidin-2-yl}-benzoesäure wird nach dem folgenden Reaktionsschema erhalten:

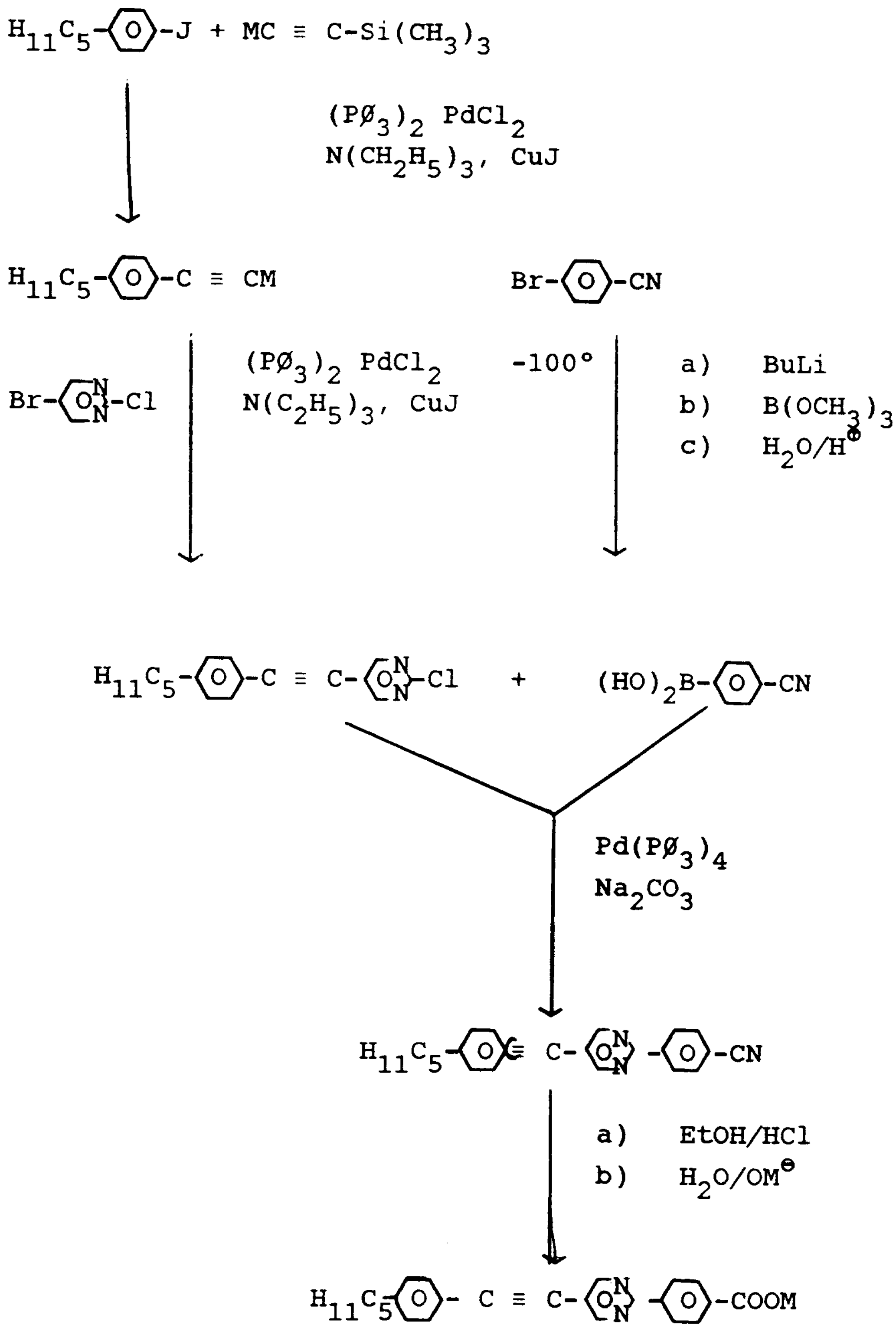


0,01 mol dieser Säure, 0,001 mol Dimethylaminopyridin und 0,01 mol (S)-2-Fluorooctanol werden in 15 ml Dichlormethan vorgelegt, bei 10° unter Rühren eine Lösung von 0,01 mol Dicyclohexylcarbodiimid

zugetropft und anschließend 15 Stunden bei Raumtemperatur nachgerührt. Man filtriert über Kieselgel ab, verdampft das Lösungsmittel und erhält als Rückstand [(S)-2-Fluoroctyl]-4-{5-[2-(4-Pentylphenyl)-ethinyl]-pyrimidin-2-yl}-benzoat.

Beispiel 5

Durch Hydrieren der in Beispiel 4 erhaltenen Verbindung mit Wasserstoff unter Normaldruck bei Raumtemperatur und Palladiumkohle als Katalysator erhält man [(S)-2-Fluoroctyl]-4-{5-12-(4-Pentylphenyl)-ethyl]-pyrimidin-2-yl}-benzoat.

Beispiel 1

Man kühlt eine Lösung von 0,1 mol 5-Heptyl-2-(4-2-hydroxy-5-oxaoctyloxy)-phenyl)-pyrimidin (hergestellt durch Erhitzen von optisch aktivem 1,2-Epoxy-5-oxaoctan, erhältlich aus Äpfelsäure, mit 5-Heptyl-2-(p-hydroxyphenyl)pyrimidin in Gegenwart von trockenem Kaliumcarbonat und Methylethylketon als Lösungsmittel) in Methylchlorid auf -40 °C und gibt dazu unter Feuchtigkeitsausschluß tropfenweise 0,11 mol DAST. Anschließend rührt man das Reaktionsgemisch unter langsamem Erwärmen auf Raumtemperatur 12 Stunden. Dann hydrolysiert man unter Eiskühlung und wäscht das Reaktionsgemisch mit verdünnter Natronlauge und mehrfach mit Wasser. Nach dem Trocknen über Magnesiumsulfat wird das Lösungsmittel am Rotationsverdampfer entfernt und das Rohprodukt chromatographisch und durch Kristallisation gereinigt. Man erhält optisch aktives 5-Heptyl-2-(4-(2-fluor-5-oxaoctyloxy)-phenyl)-pyrimidin, K 41 $S_A$ (35) I.

Analog erhält man
5-Heptyl-2-(4-(2-fluor-4-oxadecyloxy)-phenyl)-pyrimidin, K 74 I

Beispiel 2

Zu einer Lösung von optisch aktiver 2-Fluor-5-oxaoctansäure (erhältlich durch Ringöffnung von optisch aktivem 1,2-Epoxy-5-oxaoctan mit Pyridin/HF und Oxidation des Alkohols zur Säure) und Octyloxybiphenylol sowie einer katalytischen Menge DMAP in Methylenchlorid gibt man unter Eiskühlung eine Lösung von 0,11 mol DCC in Methylenchlorid. Man läßt das Reaktionsgemisch 12 Stunden bei Raumtemperatur rühren, entfernt dann den Niederschlag durch Filtration und arbeitet das Filtrat wie üblich auf. Das Produkt wird durch Kristallisation und Chromatographie gereinigt. Man erhält optisch aktives 4'-Octyloxy-4-(2-fluor-5-oxaoctanoyloxy)-biphenyl.

Beispiel 3

Zu einer Lösung von 0,1 mol 2-(p-Octyloxyphenyl)-5-(3-hydroxy-6-oxanonyl)pyridin (erhältlich durch Umsetzung von 2-(p-Octyloxyphenyl)-5-methylpyridin mit LDA bei -40 °C und optisch aktivem 1,2-Epoxy-5-oxaoctan) in Methylenchlorid gibt man bei -30 °C 0,11 mol DAST und erwärmt dann langsam das Reaktionsgemisch auf Raumtemperatur. Nach 12 Stunden arbeitet man wie üblich auf und reinigt das Produkt durch Kristallisation. Man erhält optisch aktives 2-(p-Octyloxyphenyl)-5-(3-fluor-6-oxanonyl)-pyridin.

Beispiel 4

Zu einer Lösung von 0,1 mol des durch Umsetzung von 2-p-Octyloxyphenyl-5-methylpyridin mit optisch aktivem 1,2-Epoxy-4-oxanonan in Gegenwart von LDA erhaltenen Hydroxypyridins in Methylenchlorid gibt man unter Feuchtigkeitsausschluß bei -40 °C tropfenweise eine Lösung von 0,11 mol DAST in Methylenchlorid. Anschließend läßt man das Reaktionsgemisch sich langsam auf Raumtemperatur erwärmen und rührt dann 12 Stunden. Man hydrolysiert das Reaktionsgemisch und wäscht die organische Phase mit verdünnter Natronlauge und mit Wasser neutral. Die organische Phase wird mit Magnesiumsulfat getrocknet, das Lösungsmittel abgezogen und der Rückstand durch Kristallisation und Chromatographie gereinigt. Man erhält optisch aktives 2-(p-Octyloxyphenyl)-5-(3-fluor-5-oxadecyl)-pyridin.

Beispiel 5

Unter Feuchtigkeitsausschluß und $N_2$-Atmosphäre gibt man zu einer Lösung von 90 ml THF (Tetrahydrofuran) und 15,6 ml Diisopropylamin 70 ml einer Lösung von n-BuLi in Hexan bei etwa -40 °C und dazu dann bei der gleichen Temperatur 29,7 g 2-p-Octyloxyphenyl-5-methylpyridin gelöst in 100 ml THF. Man

rührt das Reaktionsgemisch 30 Minuten bei -10 °C und gibt dazu dann 12,8 g optisch aktives 3-Fluor-1-jod-n-octan gelöst in 20 ml THF. Man rührt anschließend 3 Stunden bei Raumtemperatur und arbeitet dann wie üblich auf. Man erhält optisch aktives 2-p-Octyloxyphenyl-5-(4-fluoroctyl)-pyridin.

Analog werden hergestellt:

2-p-Octyloxyphenyl-5-(4-fluor-pentyl)-pyridin
2-p-Octyloxyphenyl-5-(4-fluor-hexyl)-pyridin
2-p-Octyloxyphenyl-5-(4-fluor-heptyl)-pyridin
2-p-Octyloxyphenyl-5-(4-fluor-octyl)-pyridin
2-p-Octyloxyphenyl-5-(4-fluor-decyl)-pyridin
2-p-Octyloxyphenyl-5-(4-fluor-undecyl)-pyridin
2-p-Octyloxyphenyl-5-(4-fluor-dodecyl)-pyridin
2-p-Hexyloxyphenyl-5-(4-fluor-pentyl)-pyridin
2-p-Hexyloxyphenyl-5-(4-fluor-hexyl)-pyridin
2-p-Hexyloxyphenyl-5-(4-fluor-heptyl)-pyridin
2-p-Hexyloxyphenyl-5-(4-fluor-octyl)-pyridin
2-p-Hexyloxyphenyl-5-(4-fluor-nonyl)-pyridin
2-p-Hexyloxyphenyl-5-(4-fluor-decyl)-pyridin
2-p-Hexyloxyphenyl-5-(4-fluor-undecyl)-pyridin
2-p-Hexyloxyphenyl-5-(4-fluor-dodecyl)-pyridin
2-p-Heptyloxyphenyl-5-(4-fluor-pentyl)-pyridin
2-p-Heptyloxyphenyl-5-(4-fluor-hexyl)-pyridin
2-p-Heptyloxyphenyl-5-(4-fluor-heptyl)-pyridin
2-p-Heptyloxyphenyl-5-(4-fluor-octyl)-pyridin
2-p-Heptyloxyphenyl-5-(4-fluor-nonyl)-pyridin
2-p-Heptyloxyphenyl-5-(4-fluor-decyl)-pyridin
2-p-Heptyloxyphenyl-5-(4-fluor-undecyl)-pyridin
2-p-Heptyloxyphenyl-5-(4-fluor-dodecyl)-pyridin
2-p-Nonyloxyphenyl-5-(4-fluor-pentyl)-pyridin
2-p-Nonyloxyphenyl-5-(4-fluor-hexyl)-pyridin
2-p-Nonyloxyphenyl-5-(4-fluor-heptyl)-pyridin
2-p-Nonyloxyphenyl-5-(4-fluor-octyl)-pyridin
2-p-Nonyloxyphenyl-5-(4-fluor-nonyl)-pyridin
2-p-Nonyloxyphenyl-5-(4-fluor-decyl)-pyridin
2-p-Nonyloxyphenyl-5-(4-fluor-undecyl)-pyridin
2-p-Nonyloxyphenyl-5-(4-fluor-dodecyl)-pyridin
2-p-Decyloxyphenyl-5-(4-fluor-pentyl)-pyridin
2-p-Decyloxyphenyl-5-(4-fluor-hexyl)-pyridin
2-p-Decyloxyphenyl-5-(4-fluor-heptyl)-pyridin
2-p-Decyloxyphenyl-5-(4-fluor-octyl)-pyridin
2-p-Decyloxyphenyl-5-(4-fluor-nonyl)-pyridin
2-p-Decyloxyphenyl-5-(4-fluor-decyl)-pyridin
2-p-Decyloxyphenyl-5-(4-fluor-undecyl)-pyridin
2-p-Decyloxyphenyl-5-(4-fluor-dodecyl)-pyridin
2-(3-Fluor-4-Octyloxyphenyl-5-(4-fluor-pentyl)-pyridin
2-(3-Fluor-4-Octyloxyphenyl-5-(4-fluor-hexyl)-pyridin
2-(3-Fluor-4-Octyloxyphenyl-5-(4-fluor-heptyl)-pyridin
2-(3-Fluor-4-Octyloxyphenyl-5-(4-fluor-nonyl)-pyridin
2-(3-Fluor-4-Octyloxyphenyl-5-(4-fluor-decyl)-pyridin
2-(3-Fluor-4-Octyloxyphenyl-5-(4-fluor-undecyl)-pyridin
2-(3-Fluor-4-Octyloxyphenyl-5-(4-fluor-dodecyl)-pyridin
2-(2,3-Difluor-4-Octyloxyphenyl-5-(4-fluor-pentyl)-pyridin
2-(2,3-Difluor-4-Octyloxyphenyl-5-(4-fluor-hexyl)-pyridin
2-(2,3-Difluor-4-Octyloxyphenyl-5-(4-fluor-heptyl)-pyridin
2-(2,3-Difluor-4-Octyloxyphenyl-5-(4-fluor-octyl)-pyridin
2-(2,3-Difluor-4-Octyloxyphenyl-5-(4-fluor-nonyl)-pyridin
2-(2,3-Difluor-4-Octyloxyphenyl-5-(4-fluor-decyl)-pyridin
2-(2,3-Difluor-4-Octyloxyphenyl-5-(4-fluor-undecyl)-pyridin
2-(2,3-Difluor-4-Octyloxyphenyl-5-(4-fluor-dodecyl)-pyridin

2-p-Heptyloxyphenyl-5-(3-fluorpentyl)-pyridin
2-p-Heptyloxyphenyl-5-(3-fluorhexyl)-pyridin
2-p-Heptyloxyphenyl-5-(3-fluorheptyl)-pyridin
2-p-Heptyloxyphenyl-5-(3-fluoroctyl)-pyridin
2-p-Heptyloxyphenyl-5-(3-fluornonyl)-pyridin, K 67 $S_B$ 71 $S_A$ 87 I
2-p-Heptyloxyphenyl-5-(3-fluordecyl)-pyridin
2-p-Heptyloxyphenyl-5-(3-fluorundecyl)-pyridin
2-p-Heptyloxyphenyl-5-(3-fluordodecyl)-pyridin
2-p-Nonyloxyphenyl-5-(3-fluorpentyl)-pyridin
2-p-Nonyloxyphenyl-5-(3-fluorhexyl)-pyridin
2-p-Nonyloxyphenyl-5-(3-fluorheptyl)-pyridin
2-p-Nonyloxyphenyl-5-(3-fluoroctyl)-pyridin
2-p-Nonyloxyphenyl-5-(3-fluornonyl)pyridin, K 73 $S_I$ 74 $S_c$ 87 $S_A$ 89 I
2-p-Nonyloxyphenyl-5-(3-fluordecyl)-pyridin
2-p-Nonyloxyphenyl-5-(3-fluorundecyl)-pyridin
2-p-Nonyloxyphenyl-5-(3-fluordodecyl)-pyridin
2-p-Octyloxyphenyl-5-(3-fluorpentyl)-pyridin
2-p-Octyloxyphenyl-5-(3-fluorhexyl)-pyridin
2-p-Octyloxyphenyl-5-(3-fluorheptyl)-pyridin
2-p-Octyloxyphenyl-5-(3-fluoroctyl)-pyridin
2-p-Octyloxyphenyl-5-(3-fluornonyl)-pyridin, K 66 $S_I$ 73 $S_c$ 86 $S_A$ 90 I
2-p-Octyloxyphenyl-5-(3-fluordecyl)-pyridin
2-p-Octyloxyphenyl-5-(3-fluorundecyl)-pyridin
2-p-Octyloxyphenyl-5-(3-fluordodecyl)-pyridin

Beispiel 6

Herstellung von 5-Decyl-2-(p-(3-fluordecyl-1-oxy)-phenyl)pyrimidin.

Unter Feuchtigkeitsausschluß gibt man unter Eiskühlung zu einem Gemisch aus 0,1 mol 5-Decyl-2-p-hydroxyphenylpyrimidin, 0,105 mol optisch aktivem 3-Fluor-1-decanol und 0,1 mol Triphenylphosphin in THF tropfenweise 0,1 mol Diethylazodicarboxylat so zu, daß die Reaktionstemperatru 10 °C nicht überschritten wird. Anschließend läßt man das Reaktionsgemisch sich langsam auf Raumtemperatur erwärmen und rührt 48 Stunden. Im Vakuum wird dann das Lösungsmittel abgezogen, der Rückstand mit 3 x 100 ml Toluol extrahiert und die Toluolphase über Kieselgel gesäult. Nach dem Abdampfen des Lösungsmittels wird das Produkt durch Kristallisation gereinigt, K 56 $S_c$ 60 $S_A$ 70 I.

Auf entsprechende Weise werden 5-Decyl-2-p-hydroxyphenylpyridin, 4-Octyloxy-2,3-difluor-4'-hydroxybiphenyl, 4-Octyloxy-4'-hydroxy-2',4'-difluorbiphenyl und 4-Octyloxy-4'-hydroxybiphenyl mit optisch aktivem 3-Fluor-1-decanol nach Mitsunobu (Synthesis 1, 1981.) verethert. das Reaktionsgemisch langsam erwärmen bis eine klare Lösung entsteht und gibt dann 0,2 mol 3-Trifluormethylheptyljodid (optisch aktiv) zu. Nach 12 Stunden hydrolysiert man das Reaktionsgemisch mit gesättigter Ammoniumchlorid-Lösung und arbeitet dann wie üblich auf. Man erhält optisch aktives 2-(p-Octyloxyphenyl)-5-(4-trifluormethyl-octyl)-pyridin.

Beispiel 15

Zu 0,15 mol $POCl_3$ gibt man unter Kühlung bei maximal 30° 14,3 ml (0,185 mol) DMF und rührt 15 Minuten. Zu der Reaktionslösung gibt man 22,5 g (0,1 mol) 2-Benzyloxyacetaldehyddiethylacetal gelöst in 50 ml DMF. Anschließend wird das Reaktionsgemisch 12 Stunden lang auf 50 °C erhitzt und dann bei Raumtemperatur p-(4-Trifluormethyloctyl)-Benzamidihydrochlorid (0,1 mol) portionsweise eingetragen. Man rührt 30 Minuten und gibt dann 10 ml Triethylamin zu. Dabei steigt die Temperatur auf 80 °C bis 90 °C an. Um das Reaktionsgemisch rührfähig zu halten, wird ggf. DMF zugesetzt. Dann destilliert man bei einer Badtemperatur von max. 160 °C einen Teil des Triethylamins ab, läßt den Rückstand auf etwa 100 °C abkühlen und gibt dann 500 ml Wasser zu, säuert mit konz. HCl an. Die ausgefallenen Kristalle werden abgesaugt, gründlich mit Wasser gewaschen, getrocknet und chromatographisch gereinigt. Anschließend hydriert man unter Normaldruck mit einem Pd/C-Katalysator die Benzylgruppe ab und alkyliert die Hydroxygruppe unter den üblichen Bedingungen:

0,1 mol Hydroxypyrimidin wird in Methylethylketon gelöst und zusammen mit 0,1 mol 1-Bromoctan in Gegenwart von 0,15 mol $K_2CO_3$ 24 Stunden am Rückfluß gekocht. Dann wird wie üblich aufgearbeitet. Man erhält optisch aktives 2-p-(4-Trifluormethyl-octyl)-phenyl-5-octyloxy-pyrimidin.

Beispiel 7

4'-(4-r-cis-Cyano-4-heptylcyclohexyl)-4-(2-fluoroctyloxy)-biphenyl

3,8 g 4'-(4-r-cis-Cyano-4-heptylcyclohexyl)-biphenyl-4-ol (erhältlich aus 4'-(4-r-cis-cyano-4-heptylcyclohexyl)-4-octyloxybiphenyl hergestellt nach DE 32 31 707 durch basische Etherspaltung mit Kalium-tertiär-Butylat in N-Methylpyrrolidon bei 180 °C), 1,5 g optisch aktives 2-Fluoroctanol und 2,67 g Triphenylphosphor werden in 50 ml THF gelöst. Dazu gibt man bei etwa 10-20 °C 1,59 ml DEAD, gelöst in 10 ml THF. Anschließend rührt man das Gemisch eine Stunde bei Raumtemperatur, engt dann das Reaktionsgemisch im Vakuum ein. Der Rückstand wird mit Toluol über eine mit Kieselgel beschichtete Fritte filtriert und das Filtrat eingeengt. Nach mehrmaligem Umkristallisieren aus Essigester erhält man optisch aktives 4,-(4-r-cis-Cyano-4-heptyl-cyclohexyl)-4-(2-fluoroctyloxy)-biphenyl, K 110 $S_A$ 159 I.

Die folgenden Beispiele betreffen erfindungsgemäße flüssigkristalline Medien:

Beispiel A

Ein flüssigkristallines Medium, bestehend aus
17,0 % 2-(p-Heptyloxyphenyl)-5-nonylpyrimidin
17,0 % 2-(p-Octyloxyphenyl)-5-nonylpyrimidin
17,0 % 2-(p-Nonyloxyphenyl)-5-nonylpyrimidin
5,7 % 2-(4-Heptyloxy-2,3-difluorphenyl)-5-nonylpyrimidin
5,7 % 2-(p-Octyloxy-2,3-difluorphenyl)-5-nonylpyrimidin
5,7 % 2-(p-Nonyloxy-2,3-difluorphenyl)-5-nonylpyrimidn
17,0 % 2-(p-Hexyloxyphenyl)-5-hexyloxypyrimidin
und
15 % optisch aktivem 4'-(2-Fluoroctyloxy)-4-(4-r-cis-cyano-4-heptylcyclohexyl)-biphenyl
Zeigt $S_c^*$ 67 $S_A^*$ 72 Ch 78 I und eine Schaltzeit von 35 µs bei 20 °C und 15 V/µm

Beispiel B

Eine flüssigkristalle Basismischung (B) bestehend aus
3,3 % 2-(p-Hexyloxyphenyl)-5-heptylpyrimidin
3,3 % 2-(p-Heptyloxyphenyl)-5-heptylpyrimidin
3,3 % 2-(p-Octyloxyphenyl)-5-heptylpyrimidin
3,3 % 2-(p-Nonyloxyphenyl)-5-heptylpyrimidin
7,7 % 2-(p-Hexyloxyphenyl)-5-nonylpyrimidin
25,3 % 2-(p-Nonyloxyphenyl)-5-nonylpyrimidin
30,8 % 4'-(4-r-cis-Cyano-4-butylcyclohexyl)-4-octyloxybiphenyl
15,4 % 4'-(4-r-cis-Cyano-4-hexylcyclohexyl)-4-heptylbiphenyl und
6,6 % r-1-Cyan-cis-4-(trans-4-pentylcyclohexyl)-1-(trans-4-pentylcyclohexyl)-cyclohexan,
zeigt $S_c$ 76 $S_A$ 80 N 99 I.

Die flüssigkristalline Basismischung (B) wird mit 10 % des optisch aktiven 2-(p-Octyloxyphenyl)-5-(3-fluornonyl)-pyridin dotiert.

Die dotierte Mischung weist folgende physikalische Eigenschaften auf:

Phasenübergänge

$S_c^*$ 76 $s_A^*$ 84 Ch 97 I
Spontanpolarisation (20 °C): -11,3 nC/cm$^2$
Schaltzeit (20 °C, 15 Vµm$^{-1}$); 150 µs

Beispiel C

Die flüssigkristalline Basismischung (B) (Zusammensetzung s. Beispiel B) wird mit 10 % des optisch aktiven 2-(p-Nonyloxyphenyl)-5-(3-fluornonyl)-pyridin dotiert.

Die dotierte Mischung weist folgende physikalischen Eigenschaften auf:

Phasenübergänge

$S_C^*$ 76 $S_A^*$ 84 Ch 96 I
Spontanpolarisation (20 °C): 10,4 $nC/cm^2$
Schaltzeit (20 °C, 15 $V\mu m^{-1}$): 136 $\mu s$

Beispiel D

Die flüssigkristalline Basismischung (B) (Zusammensetzung s. Beispiel B) wird mit 10 % des optisch aktiven 2-(p-Heptyloxyphenyl)-5-(3-fluornonyl)-pyridin dotiert.

Diese Mischung weist folgende physikalischen Eigenschaften auf:

Phasenübergange:

$S_C^*$ 74 $S_A^*$ 82 Ch 94 I
Spontanpolarisation (20 °C): 10,1 $nC/cm^2$

Beispiel E

Die flüssigkristalline Basismischung (B) wird mit 10 % des optisch aktiven 2-(p-(2-Fluor-5-oxaoctyloxy)-phenyl)-5-heptylpyrimidin dotiert.

Die dotierte Mischung weist folgende physikaltischen Eigenschaften auf:

Phasenübergänge

$S_C^*$ 70 $S_A^*$ 70,5 Ch 90 I

Beispiel F

Die flüssigkristalline Basismischung (B) wird mit 10 % des optisch aktiven 2-(p-(2-Fluor-4-oxadecyloxy)-phenyl)-5-heptylpyrimidin dotiert.

Die dotierte Mischung weist folgende physikalischen Eigenschaften auf:

Phasenübergänge

$S_C^*$ 69 $S_A^*$ 73 Ch 91 I
Spontanpolarisation (20 °C): 10,6 $nC/cm^2$
Schaltzeit (20 °C, 15 $V\mu m^{-1}$): 140 $\mu s$

## Patentansprüche

**1.** Optisch aktive Verbindungen der Formel I

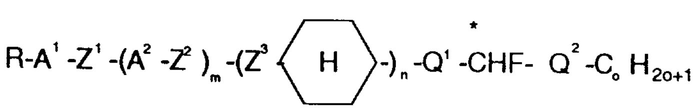

I

worin

R einen unsubstituierten, einen einfach durch -CN oder einen mindestens einfach durch Fluor oder Chlor substituierten Alkyl- oder Alkenylrest mit bis zu 15 C-Atomen, wobei in diesen Resten auch eine $CH_2$-Gruppe durch -O-,-CO-, -O-CO-, -CO-O- oder -O-CO-O- ersetzt sein kann.

$A^1$ und $A^2$ jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, 1,2,4-Thiadiazol-3,5-diyl-Rest, trans-1,4-Cyclohexylen-Rest, worin auch eine

oder zwei nicht benachbarte $CH_2$-Gruppen durch -O- und/oder -S-ersetzt sein können und/oder eine CH-Gruppe durch -C(CN)- ersetzt sein kann,

$Z^1$, $Z^2$ und $Z^3$ — jeweils unabhängig voneinander -CO-O, -O-CO-, $-CH_2O-$, - $OCH_2$- $-CH_2CH_2-$, -CH=CH-, -C≡C- oder eine Einfachbindung,

$Q^1$ — $-CH_2CH_2-$, $-CH_2CH_2CH_2-$, $-OCH_2-$, $CH_2OCH_2$-, - $OCH_2CH_2$-, -O-CO-,

$Q^2$ — -O-, -O-CO-, $-(CH_2)_3-O-$ oder eine Einfachbindung,

m — 0, 1, 2 oder 3,

n — 0 oder 1 und

o — 1 bis 9,

bedeuten,

mit den Maßgaben, daß im Falle $Q^1$ = $-OCH_2-$ oder -O-CO- einer der Ringe $A^1$ und $A^2$ trans- 1,4-Cyclohexylen, worin eine CH-Gruppe durch -C(CN)- ersetzt ist, bedeutet.

**2.** Optisch aktive Verbindungen der Formel IV

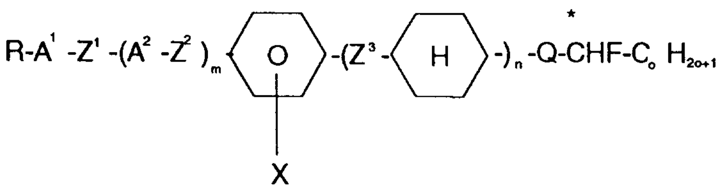

IV

worin

R — eine unsubstituierten, einen einfach durch -CN oder einen mindestens einfach durch Fluor oder Chlor substituierten Alkyl- oder Alkenylrest mit bis zu 15 C-Atomen, wobei in diesen Resten auch eine $CH_2$-Gruppe durch -O-,-CO-, -O-CO-, -CO-O- oder -O-CO-O- ersetzt sein kann.

$A^1$ und $A^2$ — jeweils unabhängig voneinander einen unsubstituierten oder durch ein oder zwei Fluoratome substituierten 1,4-Phenylen-Rest, Pyridin-2,5-diyl-Rest, Pyrimidin-2,5-diyl-Rest, Pyrazin-2,5-diyl-Rest, Pyridazin-3,6-diyl-Rest, 1,3,4-Thiadiazol-2,5-diyl-Rest, 1,2,4-Thiadiazol-3,5-diyl-Rest, trans-1,4-Cyclohexylen-Rest, worin auch eine oder zwei nicht benachbarte $CH_2$-Gruppen durch -O- und/oder -S-ersetzt sein können und/oder eine CH-Gruppe durch -C(CN)- ersetzt sein kann,

$Z^1$, $Z^2$ und $Z^3$ — jeweils unabhängig voneinander -CO-O, -O-CO-, $-CH_2O-$, - $OCH_2$- $-CH_2CH_2-$, -CH=CH-, -C≡C- oder eine Einfachbindung,

X — H oder F,

Q — $-OCH_2-$ oder $-CH_2OCH_2-$,

o — 1 bis 9,

und einer der beiden Werte

m und n 0 und der andere 0 oder 1 bedeutet,

mit den Maßgaben, daß im Falle Q = $-OCH_2-$ oder einer der Ringe $A^1$ und $A^2$ trans-1,4-Cyclohexylen, worin eine CH-Gruppe durch -C(CN)-ersetzt ist, bedeutet.

**3.** Optisch aktive Verbindungen nach Anspruch 1 oder 2,
worin

R — Pentyl, Hexyl, Heptyl, Octyl, Nonyl, Decyl, Pentoxy, Hexoxy, Heptoxy, Octoxy, Nonoxy oder Decoxy bedeutet.

**4.** Optisch aktive Verbindungen nach einem der Ansprüche 1 bis 3, worin $Z^1$ eine Einfachbindung bedeutet.

**5.** 4'-(4-r-cis-Cyano-4-heptylcyclohexyl)-4-(2-fluoroctyloxy)-biphenyl.

**6.** Chirale getiltete smektische flüssigkristalline Phase mit mindestens zwei flüssigkristallinen Komponenten, dadurch gekennzeichnet, daß sie mindestens eine Verbindung der Formeln I oder IV nach Anspruch 1 oder 2 enthält.

**7.** Chiralte getiltete Phase nach Anspruch 6, dadurch gekennzeichnet, daß sie neben einer Verbindung der Formeln I oder IV mindestens eine Verbindung der Formel VIIIj enthält,

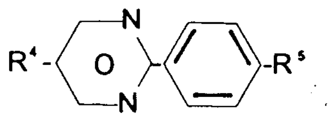

VIIIj

worin $R^4$ und $R^5$ jeweils unabhängig voneinander geradkettiges oder verzweigtes Alkyl, Alkoxy, Alkanoyloxy oder Alkoxycarbonyl mit jeweils 3 bis 12 C-Atomen bedeuten,
und/oder der Formel IXd enthält,

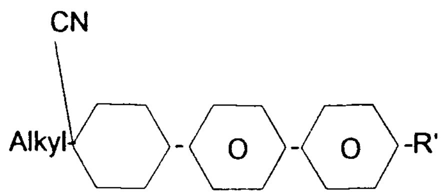

IXd

worin Alkyl eine geradkettige oder verzweigte Alkylgruppe mit 3 bis 10 C-Atomen, und R' eine geradkettige Alkyl- oder Alkoxygruppe mit 2 bis 10 C-Atomen bedeuten.

**8.** Verwendung der Verbindungen der Formeln I oder IV nach Anspruch 1 oder 2 als Komponenten flüssigkristalliner Phasen.

**9.** Elektrooptische Anzeigeelemente, dadurch gekennzeichnet, daß es als Dielektrikum eine Phase nach Anspruch 6 enthält.

**Claims**

**1.** Optically active compounds of the formula I

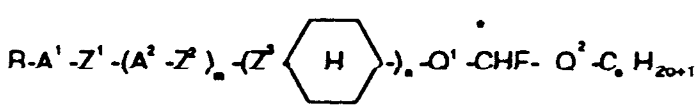

I

in which

| | |
|---|---|
| R | is an alkyl or alkenyl radical having up to 15 carbon atoms which is unsubstituted, monosubstituted by -CN or at least monosubstituted by fluorine or chlorine, it also being possible for a $CH_2$ group in these radicals to be replaced by -O-, -CO-, -O-CO-, -CO-O- or -O-CO-O-, |
| $A^1$ and $A^2$ | are each, independently of one another, a 1,4-phenylene radical, pyridine-2,5-diyl radical, pyrimidine-2,5-diyl radical, pyrazine-2,5-diyl radical, pyridazine-3,6-diyl radical, 1,3,4-thiadiazole-2,5-diyl radical, 1,2,4-thiadiazole-3,5-diyl radical or trans-1,4-cyclohexylene radical, each of which is unsubstituted or substituted by one or two fluorine atoms, and in which, in addition, one or two non-adjacent $CH_2$ groups may be replaced by -O-and/or -S-, and/or one CH group may be replaced by -C-(CN)-, |
| $Z^1$, $Z^2$ and $Z^3$ | are each, independently of one another, -CO-O-, -O-CO-, $-CH_2O-$, $-OCH_2-$, $-CH_2CH_2-$, -CH=CH-, -C≡C- or a single bond, |
| $Q^1$ | is $-CH_2CH_2-$, $-CH_2CH_2CH_2-$, $OCH_2-$, $-CH_2OCH_2-$ or $-OCH_2CH_2-$or -O-CO- |
| $Q^2$ | is -O-, -O-CO-, $-(CH_2)_3-O-$ or a single bond, |
| m | is 0, 1, 2 or 3, |
| n | is 0 or 1, and |

o is 1 to 9,

with the provisos that, in the case where $Q^1$ = $-OCH_2-$ or -O-CO-, one of the rings $A^1$ and $A^2$ is trans-1,4-cyclohexylene in which one CH group has been replaced by -C(CN)-.

**2.** Optically active compounds of the formula IV

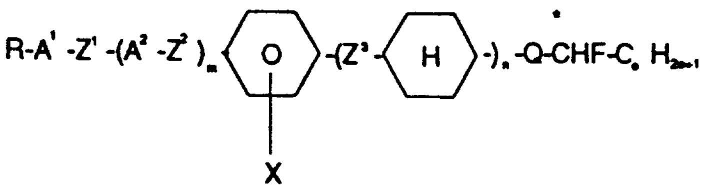

IV

in which

R is an alkyl or alkenyl radical having up to 15 carbon atoms which is unsubstituted, monosubstituted by -CN or at least monosubstituted by fluorine or chlorine, it also being possible for one $CH_2$ group in these radicals to be replaced by -O-, -CO-, -O-CO-, -CO-O- or -O-CO-O-,

$A^1$ and $A^2$ are each, independently of one another, a 1,4-phenylene radical, pyridine-2,5-diyl radical, pyrimidine-2,5-diyl radical, pyrazine-2,5-diyl radical, pyridazine-3,6-diyl radical, 1,3,4-thiadiazole-2,5-diyl radical, 1,2,4-thiadiazole-3,5-diyl radical or trans-1,4-cyclohexylene radical, each of which is unsubstituted or substituted by one or two fluorine atoms, and in which, in addition, one or two non-adjacent $CH_2$ groups may be replaced by -O- and/or -S-, and/or one CH group may be replaced by -C-(CN)-.

$Z^1$, $Z^2$ and $Z^3$ are each, independently of one another, -CO-O-, -O-CO-, $-CH_2O-$, $-OCH_2-$, $-CH_2CH_2-$, -CH=CH-, -C≡C- or a single bond,

X is H or F,

Q is $-OCH_2-$ or $-CH_2OCH_2-$,

o is 1 to 9,

and one of the two values

m and n is 0 and the other is 0 or 1,

in which

with the provisos that, in the case of Q= $-OCH_2-$ or [lacuna], one of the rings $A^1$ and $A^2$ is trans-1,4-cyclohexylene in which one CH group has been replaced by -C(CN)-.

**3.** Optically active compounds according to Claim 1 or 2,

in which

R is pentyl, hexyl, heptyl, octyl, nonyl, decyl, pentoxy, hexoxy, octoxy, nonoxy or decoxy.

**4.** Optically active compounds according to one of Claims 1 to 3, in which $Z^1$ is a single bond.

**5.** 4'-(4-r-cis-Cyano-4-heptylcyclohexyl)-4-(2-fluorooctyloxy)biphenyl.

**6.** Chiral tilted smectic liquid-crystalline phase having at least two liquid-crystalline components, characterized in that it contains at least one compound of the formulae I or IV according to Claim 1 or 2.

**7.** Chiral tilted phase according to Claim 6, characterized in that it contains, in addition to a compound I or IV, at least one compound of the formula VIIIj,

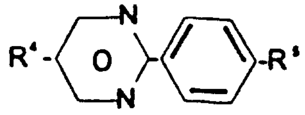

VIIIj

where $R^4$ and $R^5$ are each, independently of one another, straight-chain or branched alkyl, alkoxy,

alkanoyloxy or alkoxycarbonyl, in each case having 3 to 12 carbon atoms, and/or of the formula IXd,

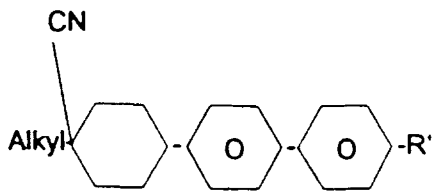

IXd

in which alkyl is a straight-chain or branched alkyl group having 3 to 10 carbon atoms, and R' is a straight-chain alkyl or alkoxy group having 2 to 10 carbon atoms.

**8.** Use of the compounds of the formulae I or IV according to Claim 1 or 2 as components of liquid-crystalline phases.

**9.** Electrooptical display elements, characterized in that it contains [sic], as dielectric, a phase according to Claim 6.

**Revendications**

**1.** Composés, possédant l'activité optique, qui répondent à la formule I

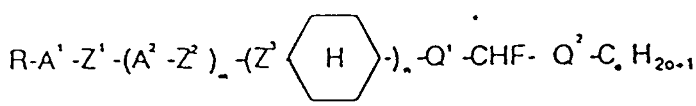

I

dans laquelle
R représente un radical alkyle ou alcényle non substitué, mono-substitué par un groupe -CN ou au moins mono-substitué par le fluor ou le chlore et contenant jusqu'à 15 atomes de carbone, et dans lequel également un groupe $CH_2$ peut être remplacé par -O-, -CO-, -O-CO-, -CO-Oou -O-CO-O-,
$A^1$ et $A^2$ représentent chacun, indépendamment l'un de l'autre, un groupe 1,4-phénylène, pyridine-2,5-diyle, pyrimidine-2,5-diyle, pyrazine-2,5-diyle, pyridazine-3,6-diyle, 1,3,4-thiadiazole-2,5-diyle, 1,2,4-thiadiazole-3,5-diyle, trans-1,4-cyclohexylène non substitué ou substitué par un ou deux atomes de fluor, et dans lequel un ou deux groupes $CH_2$ non voisins peuvent être remplacés par -O- et/ou -S- et/ou un groupe CH peut être remplacé par -C(CN)-,
$Z^1$, $Z^2$ et $Z^3$ représentent chacun, indépendamment les uns des autres -CO-O, -O-CO-, $-CH_2O-$, $-OCH_2-$ $-CH_2CH_2-$, -CH = CH-, -C≡C- ou une liaison simple,
$Q^1$ représente $-CH_2CH_2-$, $-CH_2CH_2CH_2-$, $-OCH_2-$, $CH_2OCH_2-$, $-OCH_2CH_2-$, -O-CO-,
$Q^2$ représente -O- -O-CO, $-(CH_2)_3-O-$ ou une liaison simple,
m est égal à 0, 1, 2 ou 3,
n est égal à 0 ou 1 et
o a une valeur de 1 à 9,
sous réserve que, dans le cas ou $Q^1$ = $-OCH_2-$ ou -O-CO-, l'un des cycles $A^1$ et $A^2$ est un cycle trans-1,4-cyclohexylène dans lequel un groupe CH est remplacé par -C(CN)-.

**2.** Composés possédant l'activité optique, et répondant à la formule IV

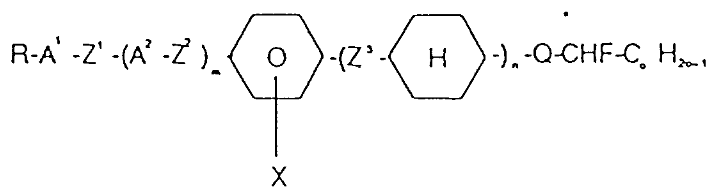

IV

dans laquelle
R représente un radical alkyle ou alcényle non substitué, monosubstitué par un groupe -CN ou au moins mono-substitué par le fluor ou le chlore et contenant jusqu'à 15 atomes de carbone, et dans lequel un groupe $CH_2$ peut également être remplacé par -O-, -CO-, -O-CO-, -CO-Oou -O-CO-O-,
$A^1$ et $A^2$ représentent chacun, indépendamment l'un de l'autre, un groupe 1,4-phénylène, pyridine-2,5-diyle, pyrimidine-2,5-diyle, pyrazine-2,5-diyle, pyridazine-3,6-diyle, 1,3,4-thiadiazole-2,5-diyle, 1,2,4-thiadiazole-3,5-diyle, trans-1,4-cyclohexylène non substitué ou substitué par un ou deux atomes de fluor et dans lequel un ou deux groupes $CH_2$ non voisins peuvent également être remplacés par -O- et/ou -S- et/ou un groupe CH peut être remplacé par -C(CN)-,
$Z^1$ , $Z^2$ et $Z^3$ représentent chacun, indépendamment les uns des autres, -CO-O, -O-CO-, $-CH_2O-$, $-OCH_2--CH_2CH_2-$, -CH=CH-, -C≡C- ou une liaison simple,
X représente H ou F ,
Q représente $-OCH_2-$ ou $-CH_2OCH_2-$,
o a une valeur de 1 à 9,
et l'un des deux indices m et n est égal à 0 et l'autre à 0 ou 1,
sous réserve que lorsque Q = $OCH_2$ ou l'un des cycles $A^1$ et $A^2$ est un cycle trans-1,4-cyclohexylène dans lequel un groupe CH est remplacé par -C(CN)-.

**3.** Composés possédant l'activité optique selon la revendication 1 ou 2, dans lesquels R représente un groupe pentyle, hexyle, heptyle, octyle, nonyle, décyle, pentoxy, hexoxy, heptoxy, octoxy, nonoxy ou décoxy.

**4.** Composés possédant l'activité optique, selon l'une des revendications 1 à 3, dans lesquels $Z^1$ représente une liaison simple.

**5.** Le 4'-(4-r-cis-cyano-4-heptylcyclohexyl)-4-(2-fluoroctyloxy)-biphényle.

**6.** Phase à cristaux liquides smectique chirale inclinée à au moins deux composants à cristaux liquides, caractérisée en ce qu'elle contient au moins un composé de formule I ou IV selon la revendication 1 ou 2.

**7.** Phase chirale inclinée selon la revendication 6, caractérisée en ce que, avec un composé de formule I ou IV, elle contient au moins un composé de formule VIIIj

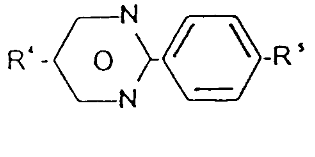

VIIIj

dans laquelle $R^4$ et $R^5$ représentent chacun, indépendamment l'un de l'autre, un radical alkyle, alcoxy, alcanoyloxy ou alcoxycarbonyle à chaîne droite ou ramifiée contenant chacun 3 à 12 atomes de carbone,
et/ou de formule IXd

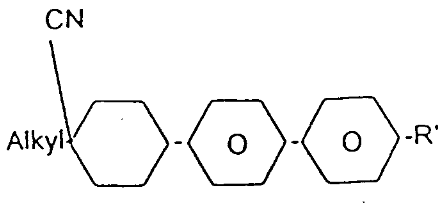

IXd

dans laquelle "Alkyl" représente un groupe alkyle à chaîne droite ou ramifiée en C3-C10 et R' un groupe alkyle ou alcoxy à chaîne droite en C2-C10.

**8.** Utilisation des composés de formule I ou IV selon la revendication 1 ou 2 en tant que composants de phases à cristaux liquides.

**9.** Eléments d'affichage électrooptiques caractérisés en ce qu'ils contiennent en tant que diélectrique une phase selon la revendication 6.